(19) Europäisches Patentamt
European Patent Office
Office européen des brevets

(11) EP 3 237 903 B1

(12) EUROPEAN PATENT SPECIFICATION

(45) Date of publication and mention of the grant of the patent:
**26.02.2020 Bulletin 2020/09**

(21) Application number: **15823296.7**

(22) Date of filing: **21.12.2015**

(51) Int Cl.:
***G01N 33/28*** (2006.01) ***C10L 1/00*** (2006.01)

(86) International application number:
**PCT/US2015/067089**

(87) International publication number:
**WO 2016/106211 (30.06.2016 Gazette 2016/26)**

# (54) METHODS FOR AUTHENTICATION AND IDENTIFICATION OF PETROLEUM PRODUCTS

VERFAHREN ZUR AUTHENTIFIZIERUNG UND/ODER IDENTIFIZIERUNG VON ERDÖLPRODUKTEN

PROCÉDÉS D'AUTHENTIFICATION ET D'IDENTIFICATION DE PRODUITS PÉTROLIERS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.12.2014 US 201462096565 P**

(43) Date of publication of application:
**01.11.2017 Bulletin 2017/44**

(73) Proprietor: **Exxonmobil Research And Engineering Company**
**Annandale, NJ 08801-0900 (US)**

(72) Inventors:
- **BLUMENFELD, Michael, L.**
  **Haddonfield, NJ 08033 (US)**
- **CAREY, James, T.**
  **Medford, NJ 08055 (US)**
- **CHRISTENSEN, Gary**
  **Wenonah, NJ 08090 (US)**
- **DIETZ, Thomas, G.**
  **Deal Island, Maryland 21821 (US)**

(74) Representative: **ExxonMobil Chemical Europe Inc.**
**IP Law Europe**
**Hermeslaan 2**
**1831 Machelen (BE)**

(56) References cited:
**EP-A1- 0 513 604**
**EP-A2- 0 327 163**
**WO-A1-99/42613**
**US-A- 5 776 713**
**US-A- 5 984 983**
**US-A1- 2004 147 413**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 237 903 B1

## Description

### FIELD

This disclosure relates to methods for authentication and identification of petroleum products. In particular, this disclosure relates to methods for authentication and identification of lubricating engine oils involving taggants that comprise one or more amide compounds (e.g., taggant arrays that comprise two or more amide compounds) and an immunoassay specific for the taggants. This disclosure also relates to petroleum products containing taggants that comprise one or more amide compounds (e.g., taggant arrays that comprise two or more amide compounds), and methods for analyzing the taggants.

### BACKGROUND

Significant problems experienced in many areas of the world and in connection with many different products is that of product counterfeiting, unauthorized distribution and sale of a product (e.g., grey market trading, parallel trading, and product diversion), as well as false liability based on product substitution.

Throughout the world, suppliers or merchants provide the products they sell with a visually distinctive appearance, packaging or labels so that customers can distinguish their products from those of others. As a result, their customers learn to associate the visually distinctive appearance with certain standards of quality, and, if they are satisfied with those standards, will buy products provided with that visually distinctive appearance in preference to others. Once customers have acquired a preference for products provided with a particular visually distinctive appearance, the suppliers or merchants become vulnerable to product counterfeiting.

Counterfeit products consist of products that are provided with a visually distinctive appearance confusingly similar to that of genuine products. Customers seeing the visually distinctive appearance provided to the counterfeit products buy these products in the expectation that they are buying genuine products.

There are many ways known of providing products with a visually distinctive appearance. In general, the visually distinctive appearance is provided either directly to the product or to an article with which the material is associated, for example a label, wrapper or container. The visually distinctive appearance may be, for example, a distinctive shape or configuration, a distinctive marking, or a combination of the two.

The material of a counterfeit product may be the same as, or different from the material of a genuine product. Often the material of the counterfeit product is the same, but of inferior quality. For instance, it is usually difficult to distinguish a chemical product having a particular chemical formula and made by one manufacturer, from the same chemical, with the same formula, but made by a different manufacturer. This is particularly so if the two manufacturers use the same production process. For this reason, it is not difficult for the unscrupulous to establish the chemical formula of an active ingredient in a composition, and the relative amounts of the various ingredients in the composition, and then pass off his own product as that of another manufacturer.

In addition to product counterfeiting, product adulteration is another major problem. Product adulteration takes place when a product is tampered with such as by dilution. An example of such a problem lies in the adulteration of lubricating oils, by addition of a counterfeiter's oil to a genuine product. Such adulteration is not only financially damaging to the oil manufacturer but the consequent lowering of performance which can occur can cause damage to the consumer and consequently harm the reputation of the genuine product.

The problems of counterfeit petroleum products are widespread and well documented. Branded products, which possess favorable properties over competitors, are imitated for commercial gain. These counterfeit products may appear visually identical to the consumer as the branded product, but may lack favorable properties afforded through the addition of proprietary chemical additives. Moreover, significant commercial gain may also occur through the adulteration of a branded product with, for example, a readily available commercial solvent. As such, the ability to distinguish a genuine product or the dilution of such from imitations is valuable. Known methods for marking petroleum products are disclosed in EP0327163, which discloses an authentication of a petroleum product with a visually undetectable marker by use of an immunoassay. EP0513604 discloses a further method of marking petroleum products with an array of taggants including amides for labelling of petroleum products. US5984983 discloses a further method of tagging petroleum products with amides.

There is a need for a method which can be used, particularly in the field, for detecting attempts at product counterfeiting and product adulteration. There is also a need for the ability, particularly in the field, to be able to identify a lubricating oil. In particular, there is a need to identify petroleum products with missing labels, or to determine whether lubricant product misapplication is responsible for an equipment failure.

## Summary

The present invention relates to methods as defined in claim 1 and 2. All numerical values within the detailed description and the claims herein are modified by "about" or "approximately" the indicated value, and take into account experimental error and variations that would be expected by a person having ordinary skill in the art.

This disclosure relates in part to methods for authentication and identification of lubricating engine oils involving taggants that comprise one or more amide compounds (e.g., taggant arrays that comprise two or more amide compounds) and an immunoassay specific for the taggant arrays.

This disclosure also relates in part to a method for authenticating a product. The method involves associating a taggant (e.g., taggant array) with a product to produce a signature product. The taggant, when associated with the signature product, is visually undetectable. The taggant comprises one or more amide compounds (e.g., a taggant array that comprises two or more amide compounds). The method also involves identifying the taggant in the signature product by an immunoassay specific for the taggant; mapping the taggant of the signature product to a batch code of the signature product; obtaining a test product to determine authenticity of the test product; identifying the presence or absence of a taggant in the test product by an immunoassay specific for the taggant; and comparing results of the immunoassay carried out on the test product with results of the immunoassay carried out on the signature product to determine authenticity of the test product.

This disclosure also relates in part to a method for identifying a product. The method involves associating a taggant (e.g., taggant array) with a product to produce a signature product. The taggant, when associated with the signature product, is visually undetectable. The taggant comprises one or more amide compounds (e.g., a taggant array that comprises two or more amide compounds). The method also involves identifying the taggant in the signature product by an immunoassay specific for the taggant; mapping the taggant of the signature product to a product code of the signature product; obtaining a test product to determine identification of the test product; identifying the presence or absence of a taggant in the test product by an immunoassay specific for the taggant; and comparing results of the immunoassay carried out on the test product with results of the immunoassay carried out on the signature product to determine identification of the test product.

It has been surprisingly found that, in accordance with this disclosure, taggants that comprise one or more amide compounds not being proteins (e.g., taggant arrays) can be used in immunoassay methods for authenticating and identifying lubricating oils. The immunoassay methods can be used, particularly in the field, for detecting attempts at product counterfeiting and product adulteration. In particular, it has been surprisingly found that, in accordance with this disclosure, amide compounds, and in particular cyclic amide compounds, show unique benefits when used as taggants in conjunction with immunoassay methods for authenticating and identifying lubricating oils.

Further, it has been surprisingly found that amide taggants show particular advantages when used in lubricating oils due to their thermal stability, oxidative stability, solubility and compatibility with additives used in lubricating oil compositions. The performance of amide compounds is set apart from other taggants used in conjunction with immunoassays, such as proteins or nitrogen containing small molecules, that do not contain amide functionality. The immunoassay methods can be used, particularly in the field, for detecting attempts at product counterfeiting and product adulteration.

Other objects and advantages of the present disclosure will become apparent from the detailed description that follows.

## BRIEF DESCRIPTION OF THE DRAWINGS

Fig. 1 shows examples of "detect" and "no detect" from immunoassay interrogation of a lubricating oil using an amide taggant in accordance with this disclosure.

Fig. 2 shows examples of a chemical bar code for determining product authenticity in accordance with this disclosure.

Fig. 3 shows an example of a chemical bar code for determining product authenticity (A-E corresponds to product authenticity) and identity (1-8 corresponds to product identity) in accordance with this disclosure.

Fig. 4 shows an illustrative authentication scheme in accordance with this disclosure.

Fig. 5 shows examples of tag molecules that can be used in lubricants and their utility in anti-counterfeiting in accordance with this disclosure.

DETAILED DESCRIPTION

In general, this disclosure involves chemical cryptography, in particular, the use of an inert marker or a variable array of inert markers for the purpose of establishing identity and authenticity of a lubricating oil, by immunoassay methods.

In particular, this disclosure uses a taggant or an array of taggants to provide unique anti-counterfeiting protection for lubricating oils. The taggant array is a series of N (where N = 1 or more) taggant molecules, each with a separate chemical identity that can be independently detected (e.g. by immunoassay) as either "present" or "absent" in the lubricant product. Each of the taggants in the array can either be included or omitted in a given batch of lubricant at manufacture, providing $2^N$ unique combinations in the array. Each combination can then be mapped to the lubricant blend code through the use of a decoder key which is closely guarded by the lubricant marketing company. When a customer purchases the lubricant, the batch code can be entered, for example, into a secure website to retrieve a diagram of the expected results. Alternatively, the customer can use their mobile phone as a bar-code reader to authenticate the lubricant. If the website and test results agree, then the customer can be assured the lubricant product is genuine.

A unique aspect of this disclosure is the ability to authenticate chemical information, i.e., the result of interrogating a lubricant using the test strip and package information (e.g., the batch # printout) through the use of a secure database. This provides improvements over either independently and prevents counterfeiters from adapting to the security measures. The lubricant marketing company can periodically alter the taggant or taggant array thereby in essence changing the chemical password. Alternatively this can be done by changing the batch codes and the database.

In general, the disclosure features a method of marking a petroleum product for authentication and/or identification in which a marker, composed of a taggant that comprises one or more amide compounds (e.g., a taggant array that comprises two or more amide compounds), is associated with the petroleum product. The marker is non-deleterious to the petroleum product, is used sparingly in the petroleum product, is soluble in water, does not interact with the petroleum product chemistry, and is robustly detectable. The presence of the marker can only be easily established by someone who knows the identity of the marker, but cannot be routinely determined by a counterfeiter or other person unfamiliar with the marker. Thus, a counterfeit and a genuine product can be distinguished by the absence of the marker in the former and the presence of the marker in the latter.

As used herein, by marker is meant a taggant that comprises one or more compounds, preferably one or more amide compounds. More preferably, the marker is a taggant array that comprises two or more amide compounds.

As used herein, by marking a product for authentication and/or identification is meant associating a marker with a product so that the source, identity, or other information about the product including production date, batch, and shelf-life may be established. Identification of a marked product can also facilitate: 1) monitoring of manufacturing or other processes, including monitoring process streams and blending controls; 2) product monitoring for security or regulatory purposes, such as marking the source country of products for customs and marking regulated substances; 3) detecting and monitoring spillages of marked materials, including the detection of residues of marked products, such as toxic wastes, organic pollutants and other chemicals; 4) tracing a product, such as marking a process chemical to monitor the rate of addition of the chemical to a system in order to optimize chemical dosage; and 5) studies of biodegradation of a compound, e.g., in soil biodegradation studies. Marking a product for identification also includes the associating a product with a particular concentration of a marker, so to facilitate the detection of product adulteration by way of dilution, concentration changes, or the addition of foreign substances.

In accordance with this disclosure, a method is provided that associates a taggant (e.g., a taggant array) with a product to produce a signature (i.e., genuine) product. The taggant, when associated with the signature product, is visually undetectable; and comprises one or more amide compounds (e.g., a taggant array that comprises two or more amide compounds). The method also identifies the taggant in the signature product by an immunoassay specific for the taggant; maps the taggant of the signature product to a batch code of the signature product; obtains a test product to determine authenticity or identity of the test product; identifies the presence or absence of a taggant in the test product by an immunoassay specific for the taggant; and compares results of the immunoassay carried out on the test product with results of the immunoassay carried out on the signature product to determine authenticity or identity of the test product.

In an embodiment, the method maps the taggant (e.g., taggant array) of the signature product to a batch code of the signature product through the use of a decoder key. The mapped taggant of the signature product to a batch code of the signature product is preferably obtained from a supplier website or database. The mapped taggant of the signature product to a batch code of the signature product is then compared with an immunoassay carried out on a purchased product to determine authenticity and/or identity of the purchased product.

The taggant (e.g., taggant array) of this disclosure is capable of being detected by immunoassay. The immunoassay is carried out using a test strip that is specific for the taggant. The test strip can be a coded test strip that can be read by a bar code reader.

In an embodiment, the test strip can include a taggant and a product identification (e.g., a taggant array and a product identification array). The test strip can preferably be a lateral flow immunoassay.

As described herein, the taggant comprises one or more amide compounds (e.g., a taggant array that comprises

two or more amide compounds). In particular, the taggant compounds are selected from aliphatic amide compounds and/or cyclic amide compounds. The one or more amide compounds include (i) one or more aliphatic amide compounds, (ii) one or more cyclic amide compounds, or (iii) a mixture of at least one aliphatic amide compound and at least one cyclic amide compound.

The aliphatic amide compounds include, for example, pyridine-3-carboxylic acid amide, N-(1-cyano-1,2-dimethylpropyl)-2-(2,4-dichlorophenoxy)propanamide, 1-benzoyl-4-propionylpiperazine, (2S)-2-{[(2S)-2-aminobutanoyl]amino}propanoic acid, 3-[decyl(dimethyl)silyl]-N-[2-(4-methylphenyl)-1-phenylethyl]propanamide, N-allyl-4,5-dimethyl-2-(trimethylsilyl)-3-thiophenecarboxamide, benzyl (1S)-2-({2-[(2-amino-2-oxoethyl)amino]-2-oxoethyl}amino)-1-benzyl-2-oxoethylcarbamate, benzyl (1S,2S)-1-({[(1R)-2-amino-1-benzyl-2-oxoethyl]amino}carbonyl)-2-hydroxypropylcarbamate, N-cyclohexyl-N-methyl-4-[(2-oxo-1,2-dihydro-6-quinolinyl)oxy]butanamide, N-[2-(1H-indol-3-yl)ethyl]tetracosanamide, N-[2-(1H-indol-3-yl)ethyl]heptadecanamide, and the like.

The cyclic amide compounds include, for example, (5S)-1-methyl-5-(3-pyridyl)pyrrolidin-2-one, 1,3-diethyl-2-thioxodihydro-4,6(1H,5H)-pyrimidinedione, N-[2-(1H-indol-3-yl)ethyl]heptadecanamide, 3-{2-[4-(6-fluoro-1,2-benzisoxazol-3-yl)-1-piperidinyl]ethyl}-2-methyl-6,7,8,9-tetrahydro-4H-pyrido[1,2-a]pyrimidin-4-one, biotinyl-N-hydroxy-succinimide, 4-ethyl-2-pyrrolidinone, 2-azaspiro[4.6]undecan-3-one, 2-azaspiro[4.4]nonan-3-one, ethyl 2-oxo-3-pyrrolidinecarboxylate, ethyl 5-oxo-3-pyrrolidinecarboxylate, N-(2-ethylhexyl)-5-norbornene-2,3-dicarboximide, 1-(3-aminophenyl)-2-pyrrolidinone, N-(2,6-dimethylphenyl)-2-(2-oxo-1-pyrrolidinyl)acetamide, (3S)-5-oxo-1-[(1S)-1-phenylethyl]-3-pyrrolidinecarboxylic acid, methyl 2-{[(2,5-dioxo-1-pyrrolidinyl)oxy]carbonyl}benzoate, 4-[3-(cyclopentyloxy)-4-methoxyphenyl]-2-pyrrolidinone, 1-{[(7-hydroxy-2-oxo-2H-chromen-3-yl)carbonyl]oxy}-2,5-pyrrolidinedione, 2-(1-hydroxyundecyl)-1-(4-nitroanilino)-6-phenyl-4a,7a-dihydro-1H-pyrrolo[3,4-b]pyridine-5,7(2H,6H)-dione, 1-methyl-2-piperidinone, 1,5-dimethyl-2-piperidinone, 1,4,4-trimethyl-2,6-piperidinedione, 4-methyl-1-undecyl-2-piperidinone, 4-methyl-1-decyl-2-piperidinone, 1-(1-adamantyl)-2-piperidinone, 3,3-(butane-1,4-diyl)bis(1,8,8-trimethyl-3-azabicyclo[3.2.1]octane-2,4-dione), tert-butyl 2,4-dioxo-1-piperidinecarboxylate, and the like.

The taggant (e.g., taggant array) is present in an amount of from about 0.05 ppm to about 20 ppm, preferably from about 0.1 ppm to about 10 ppm, and more preferably from about 0.2 ppm to about 5 ppm.

In accordance with this disclosure, a lubricating engine oil is provided having a composition comprising a lubricating oil base stock as a major component; and a taggant (e.g., a taggant array that comprises two or more amide compounds), as a minor component. The taggant array, when associated with the lubricating oil base stock, is visually undetectable. The taggant comprises one or more amide compounds (e.g., a taggant array that comprises two or more amide compounds).

As described herein, the taggant comprises one or more amide compounds. In particular, the taggant compounds are selected from aliphatic amide compounds and/or cyclic amide compounds. The one or more amide compounds include (i) one or more aliphatic amide compounds, (ii) one or more cyclic amide compounds, or (iii) a mixture of at least one aliphatic amide compound and at least one cyclic amide compound.

Illustrative aliphatic amide compounds and cyclic amide compounds are described herein. The taggant (e.g., taggant array) is present in the lubricating engine oil in an amount from about 0.05 ppm to about 20 ppm, preferably from about 0.1 ppm to about 10 ppm, and more preferably from about 0.2 ppm to about 5 ppm.

The lubricating oil base stock preferably comprises a Group I, Group II, Group III, Group IV, or Group V base oil. The lubricating oil base stock is present in an amount of from about 70 weight percent to about 95 weight percent, based on the total weight of the lubricating engine oil.

The lubricating engine oil can further include one or more of an antiwear additive, viscosity modifiers, antioxidant, detergent, dispersant, pour point depressant, corrosion inhibitor, metal deactivator, seal compatibility additive, anti-foam agent, inhibitor, and anti-rust additive.

In accordance with this disclosure, a kit is provided for authenticating and/or identifying the source of a petroleum product by the methods described herein. The kit includes one or more test strips for carrying out immunoassay, preferably lateral flow immunoassay, of petroleum products. The test strips are specific for a taggant (e.g., taggant array). The test strips can be coded test strips that can be read by a bar code reader. The test strips can also include a taggant and a product identification (e.g., a taggant array and a product identification array).

The kits of this disclosure may also include documents for comparing the result of the detection assay with that expected from a genuine product, and may comprise instructions describing the result expected of a genuine product. The documents may include, for example, a color chart, calibration table or calibration curve. The kit may comprise a sample of marked material identical to marked genuine product to be analyzed alongside the unknown sample.

The ability to provide assay testing in kit form ensures that a person in the field, such as a supplier of a product in an environment distant from the product source, can quickly check the authenticity and/or identity of the product without recourse to laboratory facilities.

It will be apparent that, since the marker compound is in such low concentrations in the petroleum product, its presence therein is not immediately apparent to someone who is unaware of the addition. Furthermore, it would not be easy for a third party to identify the marker using routine techniques and include it in a counterfeit composition. That is

because isolation and concentration of the marker relies on the use of a specific taggant of one or more amide compounds (e.g., taggant arrays that comprise two or more amide compounds), and this would not be available to anyone who was ignorant of the identity of the marker.

Petroleum products useful in this disclosure are lubricating oils.

Lubricating Oil Base Stocks

A wide range of lubricating base oils is known in the art. Lubricating base oils that are useful in the present disclosure are natural oils, mineral oils and synthetic oils, and unconventional oils (or mixtures thereof) can be used unrefined, refined, or rerefined (the latter is also known as reclaimed or reprocessed oil). Unrefined oils are those obtained directly from a natural or synthetic source and used without added purification. These include shale oil obtained directly from retorting operations, petroleum oil obtained directly from primary distillation, and ester oil obtained directly from an esterification process. Refined oils are similar to the oils discussed for unrefined oils except refined oils are subjected to one or more purification steps to improve at least one lubricating oil property. One skilled in the art is familiar with many purification processes. These processes include solvent extraction, secondary distillation, acid extraction, base extraction, filtration, and percolation. Rerefined oils are obtained by processes analogous to refined oils but using an oil that has been previously used as a feed stock.

Groups I, II, III, IV and V are broad base oil stock categories developed and defined by the American Petroleum Institute (API Publication 1509; www.API.org) to create guidelines for lubricant base oils. Group I base stocks have a viscosity index of between about 80 to 120 and contain greater than about 0.03% sulfur and/or less than about 90% saturates. Group II base stocks have a viscosity index of between about 80 to 120, and contain less than or equal to about 0.03% sulfur and greater than or equal to about 90% saturates. Group III stocks have a viscosity index greater than about 120 and contain less than or equal to about 0.03 % sulfur and greater than about 90% saturates. Group IV includes polyalphaolefins (PAO). Group V base stock includes base stocks not included in Groups I-IV. The table below summarizes properties of each of these five groups.

| | Base Oil Properties | | |
|---|---|---|---|
| | Saturates | Sulfur | Viscosity Index |
| Group I | <90 and/or | >0.03% and | ≥80 and <120 |
| Group II | ≥90 and | <0.03% and | ≥80 and <120 |
| Group III | ≥90 and | <0.03% and | ≥120 |
| Group IV | polyalphaolefins (PAO) | | |
| Group V | All other base oil stocks not included in Groups I, II, III or IV | | |

Natural oils include animal oils, vegetable oils (castor oil and lard oil, for example), and mineral oils. Animal and vegetable oils possessing favorable thermal oxidative stability can be used. Of the natural oils, mineral oils are preferred. Mineral oils vary widely as to their crude source, for example, as to whether they are paraffinic, naphthenic, or mixed paraffinic-naphthenic. Oils derived from coal or shale are also useful. Natural oils vary also as to the method used for their production and purification, for example, their distillation range and whether they are straight run or cracked, hydrorefined, or solvent extracted.

Group II and/or Group III hydroprocessed or hydrocracked base stocks, including synthetic oils such as alkyl aromatics and synthetic esters are also well known base stock oils.

Synthetic oils include hydrocarbon oil. Hydrocarbon oils include oils such as polymerized and interpolymerized olefins (polybutylenes, polypropylenes, propylene isobutylene copolymers, ethylene-olefin copolymers, and ethylene-alphaolefin copolymers, for example). Polyalphaolefin (PAO) oil base stocks are commonly used synthetic hydrocarbon oil. By way of example, PAOs derived from $C_8$, $C_{10}$, $C_{12}$, $C_{14}$ olefins or mixtures thereof may be utilized. See U.S. Patent Nos. 4,956,122; 4,827,064; and 4,827,073.

The number average molecular weights of the PAOs, which are known materials and generally available on a major commercial scale from suppliers such as ExxonMobil Chemical Company, Chevron Phillips Chemical Company, BP, and others, typically vary from about 250 to about 3,000, although PAO's may be made in viscosities up to about 150 cSt (100°C). The PAOs are typically comprised of relatively low molecular weight hydrogenated polymers or oligomers of alphaolefins which include, but are not limited to, $C_2$ to about $C_{32}$ alphaolefins with the $C_8$ to about $C_{16}$ alphaolefins, such as 1-octene, 1-decene, 1-dodecene and the like, being preferred. The preferred polyalphaolefins are poly-l-octene, poly-1-decene and poly-1-dodecene and mixtures thereof and mixed olefin-derived polyolefins. However, the dimers of

higher olefins in the range of $C_{14}$ to $C_{18}$ may be used to provide low viscosity base stocks of acceptably low volatility. Depending on the viscosity grade and the starting oligomer, the PAOs may be predominantly trimers and tetramers of the starting olefins, with minor amounts of the higher oligomers, having a viscosity range of 1.5 to 12 cSt. PAO fluids of particular use may include 3.0 cSt, 3.4 cSt, and/or 3.6 cSt and combinations thereof. Mixtures of PAO fluids having a viscosity range of 1.5 to approximately 150 cSt or more may be used if desired.

The PAO fluids may be conveniently made by the polymerization of an alphaolefin in the presence of a polymerization catalyst such as the Friedel-Crafts catalysts including, for example, aluminum trichloride, boron trifluoride or complexes of boron trifluoride with water, alcohols such as ethanol, propanol or butanol, carboxylic acids or esters such as ethyl acetate or ethyl propionate. For example the methods disclosed by U.S. Patent Nos. 4,149,178 or 3,382,291 may be conveniently used herein. Other descriptions of PAO synthesis are found in the following U.S. Patent Nos. 3,742,082; 3,769,363; 3,876,720; 4,239,930; 4,367,352; 4,413,156; 4,434,408; 4,910,355; 4,956,122; and 5,068,487. The dimers of the $C_{14}$ to $C_{18}$ olefins are described in U.S. Patent No. 4,218,330.

Other useful lubricant oil base stocks include wax isomerate base stocks and base oils, comprising hydroisomerized waxy stocks (e.g. waxy stocks such as gas oils, slack waxes, fuels hydrocracker bottoms, etc.), hydroisomerized Fischer-Tropsch waxes, Gas-to-Liquids (GTL) base stocks and base oils, and other wax isomerate hydroisomerized base stocks and base oils, or mixtures thereof. Fischer-Tropsch waxes, the high boiling point residues of Fischer-Tropsch synthesis, are highly paraffinic hydrocarbons with very low sulfur content. The hydroprocessing used for the production of such base stocks may use an amorphous hydrocracking/hydroisomerization catalyst, such as one of the specialized lube hydrocracking (LHDC) catalysts or a crystalline hydrocracking/hydroisomerization catalyst, preferably a zeolitic catalyst. For example, one useful catalyst is ZSM-48 as described in U.S. Patent No. 5,075,269. Processes for making hydrocracked/hydroisomerized distillates and hydrocracked/hydroisomerized waxes are described, for example, in U.S. Patent Nos. 2,817,693; 4,975,177; 4,921,594 and 4,897,178 as well as in British Patent Nos. 1,429,494; 1,350,257; 1,440,230 and 1,390,359. Each of the aforementioned patents is incorporated herein in their entirety. Particularly favorable processes are described in European Patent Application Nos. 464546 and 464547. Processes using Fischer-Tropsch wax feeds are described in U.S. Patent Nos. 4,594,172 and 4,943,672.

Gas-to-Liquids (GTL) base oils, Fischer-Tropsch wax derived base oils, and other wax-derived hydroisomerized (wax isomerate) base oils be advantageously used in the instant disclosure, and may have useful kinematic viscosities at 100°C of about 3 cSt to about 50 cSt, preferably about 3 cSt to about 30 cSt, more preferably about 3.5 cSt to about 25 cSt, as exemplified by GTL 4 with kinematic viscosity of about 4.0 cSt at 100°C and a viscosity index of about 141. These Gas-to-Liquids (GTL) base oils, Fischer-Tropsch wax derived base oils, and other wax-derived hydroisomerized base oils may have useful pour points of about -20°C or lower, and under some conditions may have advantageous pour points of about -25°C or lower, with useful pour points of about -30°C to about -40°C or lower. Useful compositions of Gas-to-Liquids (GTL) base oils, Fischer-Tropsch wax derived base oils, and wax-derived hydroisomerized base oils are recited in U.S. Patent Nos. 6,080,301; 6,090,989, and 6,165,949 for example.

The hydrocarbyl aromatics can be used as a base oil or base oil component and can be any hydrocarbyl molecule that contains at least about 5% of its weight derived from an aromatic moiety such as a benzenoid moiety or naphthenoid moiety, or their derivatives. These hydrocarbyl aromatics include alkyl benzenes, alkyl naphthalenes, alkyl diphenyl oxides, alkyl naphthols, alkyl diphenyl sulfides, alkylated bis-phenol A, alkylated thiodiphenol, and the like. The aromatic can be mono-alkylated, dialkylated, polyalkylated, and the like. The aromatic can be mono- or poly-functionalized. The hydrocarbyl groups can also be comprised of mixtures of alkyl groups, alkenyl groups, alkynyl, cycloalkyl groups, cycloalkenyl groups and other related hydrocarbyl groups. The hydrocarbyl groups can range from about $C_6$ up to about $C_{60}$ with a range of about $C_8$ to about $C_{20}$ often being preferred. A mixture of hydrocarbyl groups is often preferred, and up to about three such substituents may be present. The hydrocarbyl group can optionally contain sulfur, oxygen, and/or nitrogen containing substituents. The aromatic group can also be derived from natural (petroleum) sources, provided at least about 5% of the molecule is comprised of an above-type aromatic moiety. Viscosities at 100°C of approximately 3 cSt to about 50 cSt are preferred, with viscosities of approximately 3.4 cSt to about 20 cSt often being more preferred for the hydrocarbyl aromatic component. In one embodiment, an alkyl naphthalene where the alkyl group is primarily comprised of 1-hexadecene is used. Other alkylates of aromatics can be advantageously used. Naphthalene or methyl naphthalene, for example, can be alkylated with olefins such as octene, decene, dodecene, tetradecene or higher, mixtures of similar olefins, and the like. Useful concentrations of hydrocarbyl aromatic in a lubricant oil composition can be about 2% to about 25%, preferably about 4% to about 20%, and more preferably about 4% to about 15%, depending on the application.

Alkylated aromatics such as the hydrocarbyl aromatics of the present disclosure may be produced by well-known Friedel-Crafts alkylation of aromatic compounds. See Friedel-Crafts and Related Reactions, Olah, G. A. (ed.), Interscience Publishers, New York, 1963. For example, an aromatic compound, such as benzene or naphthalene, is alkylated by an olefin, alkyl halide or alcohol in the presence of a Friedel-Crafts catalyst. See Friedel-Crafts and Related Reactions, Vol. 2, part 1, chapters 14, 17, and 18, See Olah, G. A. (ed.), Interscience Publishers, New York, 1964. Many homogeneous or heterogeneous, solid catalysts are known to one skilled in the art. The choice of catalyst depends on

the reactivity of the starting materials and product quality requirements. For example, strong acids such as $AlCl_3$, $BF_3$, or HF may be used. In some cases, milder catalysts such as $FeCl_3$ or $SnCl_4$ are preferred. Newer alkylation technology uses zeolites or solid super acids.

Esters comprise a useful base stock. Additive solvency and seal compatibility characteristics may be secured by the use of esters such as the esters of dibasic acids with monoalkanols and the polyol esters of monocarboxylic acids. Esters of the former type include, for example, the esters of dicarboxylic acids such as phthalic acid, succinic acid, alkyl succinic acid, alkenyl succinic acid, maleic acid, azelaic acid, suberic acid, sebacic acid, fumaric acid, adipic acid, linoleic acid dimer, malonic acid, alkyl malonic acid, alkenyl malonic acid, etc., with a variety of alcohols such as butyl alcohol, hexyl alcohol, dodecyl alcohol, 2-ethylhexyl alcohol, etc. Specific examples of these types of esters include dibutyl adipate, di(2-ethylhexyl) sebacate, di-n-hexyl fumarate, dioctyl sebacate, diisooctyl azelate, diisodecyl azelate, dioctyl phthalate, didecyl phthalate, dieicosyl sebacate, etc.

Particularly useful synthetic esters are those which are obtained by reacting one or more polyhydric alcohols, preferably the hindered polyols (such as the neopentyl polyols, e.g., neopentyl glycol, trimethylol ethane, 2-methyl-2-propyl-1,3-propanediol, trimethylol propane, pentaerythritol and dipentaerythritol) with alkanoic acids containing at least about 4 carbon atoms, preferably $C_5$ to $C_{30}$ acids such as saturated straight chain fatty acids including caprylic acid, capric acid, lauric acid, myristic acid, palmitic acid, stearic acid, arachic acid, and behenic acid, or the corresponding branched chain fatty acids or unsaturated fatty acids such as oleic acid, or mixtures of any of these materials.

Suitable synthetic ester components include the esters of trimethylol propane, trimethylol butane, trimethylol ethane, pentaerythritol and/or dipentaerythritol with one or more monocarboxylic acids containing from about 5 to about 10 carbon atoms. These esters are widely available commercially, for example, the Mobil P-41 and P-51 esters of ExxonMobil Chemical Company.

Also useful are esters derived from renewable material such as coconut, palm, rapeseed, soy, sunflower and the like. These esters may be monoesters, di-esters, polyol esters, complex esters, or mixtures thereof. These esters are widely available commercially, for example, the Mobil P-51 ester of ExxonMobil Chemical Company.

Engine oil formulations containing renewable esters are included in this disclosure. For such formulations, the renewable content of the ester is typically greater than about 70 weight percent, preferably more than about 80 weight percent and most preferably more than about 90 weight percent.

Other useful fluids of lubricating viscosity include non-conventional or unconventional base stocks that have been processed, preferably catalytically, or synthesized to provide high performance lubrication characteristics.

Non-conventional or unconventional base stocks/base oils include one or more of a mixture of base stock(s) derived from one or more Gas-to-Liquids (GTL) materials, as well as isomerate/isodewaxate base stock(s) derived from natural wax or waxy feeds, mineral and or non-mineral oil waxy feed stocks such as slack waxes, natural waxes, and waxy stocks such as gas oils, waxy fuels hydrocracker bottoms, waxy raffinate, hydrocrackate, thermal crackates, or other mineral, mineral oil, or even non-petroleum oil derived waxy materials such as waxy materials received from coal liquefaction or shale oil, and mixtures of such base stocks.

GTL materials are materials that are derived via one or more synthesis, combination, transformation, rearrangement, and/or degradation/deconstructive processes from gaseous carbon-containing compounds, hydrogen-containing compounds and/or elements as feed stocks such as hydrogen, carbon dioxide, carbon monoxide, water, methane, ethane, ethylene, acetylene, propane, propylene, propyne, butane, butylenes, and butynes. GTL base stocks and/or base oils are GTL materials of lubricating viscosity that are generally derived from hydrocarbons; for example, waxy synthesized hydrocarbons, that are themselves derived from simpler gaseous carbon-containing compounds, hydrogen-containing compounds and/or elements as feed stocks. GTL base stock(s) and/or base oil(s) include oils boiling in the lube oil boiling range (1) separated/fractionated from synthesized GTL materials such as, for example, by distillation and subsequently subjected to a final wax processing step which involves either or both of a catalytic dewaxing process, or a solvent dewaxing process, to produce lube oils of reduced/low pour point; (2) synthesized wax isomerates, comprising, for example, hydrodewaxed or hydroisomerized cat and/or solvent dewaxed synthesized wax or waxy hydrocarbons; (3) hydrodewaxed or hydroisomerized cat and/or solvent dewaxed Fischer-Tropsch (F-T) material (i.e., hydrocarbons, waxy hydrocarbons, waxes and possible analogous oxygenates); preferably hydrodewaxed or hydroisomerized/followed by cat and/or solvent dewaxing dewaxed F-T waxy hydrocarbons, or hydrodewaxed or hydroisomerized/followed by cat (or solvent) dewaxing dewaxed, F-T waxes, or mixtures thereof.

GTL base stock(s) and/or base oil(s) derived from GTL materials, especially, hydrodewaxed or hydroisomerized/followed by cat and/or solvent dewaxed wax or waxy feed, preferably F-T material derived base stock(s) and/or base oil(s), are characterized typically as having kinematic viscosities at 100°C of from about 2 $mm^2/s$ to about 50 $mm^2/s$ (ASTM D445). They are further characterized typically as having pour points of -5°C to about -40°C or lower (ASTM D97). They are also characterized typically as having viscosity indices of about 80 to about 140 or greater (ASTM D2270).

In addition, the GTL base stock(s) and/or base oil(s) are typically highly paraffinic (>90% saturates), and may contain mixtures of monocycloparaffins and multicycloparaffins in combination with non-cyclic isoparaffins. The ratio of the naphthenic (i.e., cycloparaffin) content in such combinations varies with the catalyst and temperature used. Further,

GTL base stock(s) and/or base oil(s) typically have very low sulfur and nitrogen content, generally containing less than about 10 ppm, and more typically less than about 5 ppm of each of these elements. The sulfur and nitrogen content of GTL base stock(s) and/or base oil(s) obtained from F-T material, especially F-T wax, is essentially nil. In addition, the absence of phosphorous and aromatics make this materially especially suitable for the formulation of low SAP products.

The term GTL base stock and/or base oil and/or wax isomerate base stock and/or base oil is to be understood as embracing individual fractions of such materials of wide viscosity range as recovered in the production process, mixtures of two or more of such fractions, as well as mixtures of one or two or more low viscosity fractions with one, two or more higher viscosity fractions to produce a blend wherein the blend exhibits a target kinematic viscosity.

The GTL material, from which the GTL base stock(s) and/or base oil(s) is/are derived is preferably an F-T material (i.e., hydrocarbons, waxy hydrocarbons, wax).

Base oils for use in the formulated lubricating oils useful in the present disclosure are any of the variety of oils corresponding to API Group I, Group II, Group III, Group IV, and Group V oils and mixtures thereof, preferably API Group II, Group III, Group IV, and Group V oils and mixtures thereof, more preferably the Group III to Group V base oils due to their exceptional volatility, stability, viscometric and cleanliness features. Minor quantities of Group I stock, such as the amount used to dilute additives for blending into formulated lube oil products, can be tolerated but should be kept to a minimum, i.e. amounts only associated with their use as diluent/carrier oil for additives used on an "as-received" basis. Even in regard to the Group II stocks, it is preferred that the Group II stock be in the higher quality range associated with that stock, i.e. a Group II stock having a viscosity index in the range $100 < VI < 120$.

The base oil constitutes the major component of the engine oil lubricant composition of the present disclosure and typically is present in an amount ranging from about 50 to about 99 weight percent, preferably from about 70 to about 95 weight percent, and more preferably from about 85 to about 95 weight percent, based on the total weight of the composition. The base oil may be selected from any of the synthetic or natural oils typically used as crankcase lubricating oils for spark-ignited and compression-ignited engines. The base oil conveniently has a kinematic viscosity, according to ASTM standards, of about 2.5 cSt to about 12 cSt (or $mm^2$ /s) at 100°C and preferably of about 2.5 cSt to about 9 cSt (or $mm^2$ /s) at 100° C. Mixtures of synthetic and natural base oils may be used if desired. Bi-modal mixtures of Group I, II, III, IV, and/or V base stocks may be used if desired.

## Additives

The formulated lubricating oil useful in the present disclosure may additionally contain one or more of commonly used lubricating oil performance additives including but not limited to detergents, antiwear additives, dispersants, viscosity modifiers, corrosion inhibitors, rust inhibitors, metal deactivators, extreme pressure additives, anti-seizure agents, wax modifiers, viscosity modifiers, fluid-loss additives, seal compatibility agents, lubricity agents, antistaining agents, chromophoric agents, defoamants, demulsifiers, emulsifiers, densifiers, wetting agents, gelling agents, tackiness agents, colorants, and others. For a review of many commonly used additives, see Klamann in Lubricants and Related Products, Verlag Chemie, Deerfield Beach, FL; ISBN 0-89573-177-0. Reference is also made to "Lubricant Additives" by M. W. Ranney, published by Noyes Data Corporation of Parkridge, NJ (1973); see also U.S. Patent No. 7,704,930, the disclosure of which is incorporated herein in its entirety. These additives are commonly delivered with varying amounts of diluent oil, that may range from 5 weight percent to 50 weight percent.

The additives useful in this disclosure do not have to be soluble in the lubricating oils. Insoluble additives such as zinc stearate in oil can be dispersed in the lubricating oils of this disclosure.

The types and quantities of performance additives used in combination with the instant disclosure in lubricant compositions are not limited by the examples shown herein as illustrations.

## Detergents

Illustrative detergents useful in this disclosure include, for example, alkali metal detergents, alkaline earth metal detergents, or mixtures of one or more alkali metal detergents and one or more alkaline earth metal detergents. A typical detergent is an anionic material that contains a long chain hydrophobic portion of the molecule and a smaller anionic or oleophobic hydrophilic portion of the molecule. The anionic portion of the detergent is typically derived from an organic acid such as a sulfur acid, carboxylic acid (e.g., salicylic acid), phosphorous acid, phenol, or mixtures thereof. The counterion is typically an alkaline earth or alkali metal. The detergent can be overbased as described herein.

The detergent is preferably a metal salt of an organic or inorganic acid, a metal salt of a phenol, or mixtures thereof. The metal is preferably selected from an alkali metal, an alkaline earth metal, and mixtures thereof. The organic or inorganic acid is selected from an aliphatic organic or inorganic acid, a cycloaliphatic organic or inorganic acid, an aromatic organic or inorganic acid, and mixtures thereof.

The metal is preferably selected from an alkali metal, an alkaline earth metal, and mixtures thereof. More preferably, the metal is selected from calcium (Ca), magnesium (Mg), and mixtures thereof.

The organic acid or inorganic acid is preferably selected from a sulfur acid, a carboxylic acid, a phosphorus acid, and mixtures thereof.

Preferably, the metal salt of an organic or inorganic acid or the metal salt of a phenol comprises calcium phenate, calcium sulfonate, calcium salicylate, magnesium phenate, magnesium sulfonate, magnesium salicylate, an overbased detergent, and mixtures thereof.

Salts that contain a substantially stochiometric amount of the metal are described as neutral salts and have a total base number (TBN, as measured by ASTM D2896) of from 0 to 80. Many compositions are overbased, containing large amounts of a metal base that is achieved by reacting an excess of a metal compound (a metal hydroxide or oxide, for example) with an acidic gas (such as carbon dioxide). Useful detergents can be neutral, mildly overbased, or highly overbased. These detergents can be used in mixtures of neutral, overbased, highly overbased calcium salicylate, sulfonates, phenates and/or magnesium salicylate, sulfonates, phenates. The TBN ranges can vary from low, medium to high TBN products, including as low as 0 to as high as 600. Mixtures of low, medium, high TBN can be used, along with mixtures of calcium and magnesium metal based detergents, and including sulfonates, phenates, salicylates, and carboxylates. A detergent mixture with a metal ratio of 1, in conjunction of a detergent with a metal ratio of 2, and as high as a detergent with a metal ratio of 5, can be used. Borated detergents can also be used.

Alkaline earth phenates are another useful class of detergent. These detergents can be made by reacting alkaline earth metal hydroxide or oxide (CaO, $Ca(OH)_2$, BaO, $Ba(OH)_2$, MgO, $Mg(OH)_2$, for example) with an alkyl phenol or sulfurized alkylphenol. Useful alkyl groups include straight chain or branched $C_1$-$C_{30}$ alkyl groups, preferably, $C_4$-$C_{20}$ or mixtures thereof. Examples of suitable phenols include isobutylphenol, 2-ethylhexylphenol, nonylphenol, dodecyl phenol, and the like. It should be noted that starting alkylphenols may contain more than one alkyl substituent that are each independently straight chain or branched and can be used from 0.5 to 6 weight percent. When a non-sulfurized alkylphenol is used, the sulfurized product may be obtained by methods well known in the art. These methods include heating a mixture of alkylphenol and sulfurizing agent (including elemental sulfur, sulfur halides such as sulfur dichloride, and the like) and then reacting the sulfurized phenol with an alkaline earth metal base.

In accordance with this disclosure, metal salts of carboxylic acids are preferred detergents. These carboxylic acid detergents may be prepared by reacting a basic metal compound with at least one carboxylic acid and removing free water from the reaction product. These compounds may be overbased to produce the desired TBN level. Detergents made from salicylic acid are one preferred class of detergents derived from carboxylic acids. Useful salicylates include long chain alkyl salicylates. One useful family of compositions is of the formula

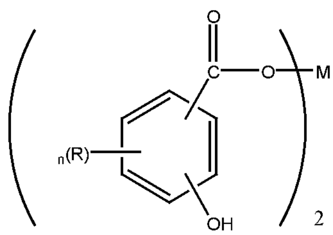

where R is an alkyl group having 1 to about 30 carbon atoms, n is an integer from 1 to 4, and M is an alkaline earth metal. Preferred R groups are alkyl chains of at least $C_{11}$, preferably $C_{13}$ or greater. R may be optionally substituted with substituents that do not interfere with the detergent's function. M is preferably, calcium, magnesium, or barium. More preferably, M is calcium.

Hydrocarbyl-substituted salicylic acids may be prepared from phenols by the Kolbe reaction (see U.S. Patent No. 3,595,791). The metal salts of the hydrocarbyl-substituted salicylic acids may be prepared by double decomposition of a metal salt in a polar solvent such as water or alcohol.

Alkaline earth metal phosphates are also used as detergents and are known in the art.

Detergents may be simple detergents or what is known as hybrid or complex detergents. The latter detergents can provide the properties of two detergents without the need to blend separate materials. See U.S. Patent No. 6,034,039.

Preferred detergents include calcium sulfonates, magnesium sulfonates, calcium salicylates, magnesium salicylates, calcium phenates, magnesium phenates, and other related components (including borated detergents), and mixtures thereof. Preferred mixtures of detergents include magnesium sulfonate and calcium salicylate, magnesium sulfonate and calcium sulfonate, magnesium sulfonate and calcium phenate, calcium phenate and calcium salicylate, calcium phenate and calcium sulfonate, calcium phenate and magnesium salicylate, calcium phenate and magnesium phenate. Overbased detergents are also preferred.

The detergent concentration in the lubricating oils of this disclosure can range from about 0.5 to about 6.0 weight percent, preferably about 0.6 to 5.0 weight percent, and more preferably from about 0.8 weight percent to about 4.0 weight percent, based on the total weight of the lubricating oil.

As used herein, the detergent concentrations are given on an "as delivered" basis. Typically, the active detergent is delivered with a process oil. The "as delivered" detergent typically contains from about 20 weight percent to about 100 weight percent, or from about 40 weight percent to about 60 weight percent, of active detergent in the "as delivered" detergent product.

Antiwear Additives

Illustrative antiwear additives useful in this disclosure include, for example, metal salts of a carboxylic acid. The metal is selected from a transition metal and mixtures thereof. The carboxylic acid is selected from an aliphatic carboxylic acid, a cycloaliphatic carboxylic acid, an aromatic carboxylic acid, and mixtures thereof.

The metal is preferably selected from a Group 10, 11 and 12 metal, and mixtures thereof. The carboxylic acid is preferably an aliphatic, saturated, unbranched carboxylic acid having from about 8 to about 26 carbon atoms, and mixtures thereof.

The metal is preferably selected from nickel (Ni), palladium (Pd), platinum (Pt), copper (Cu), silver (Ag), gold (Au), zinc (Zn), cadium (Cd), mercury (Hg), and mixtures thereof.

The carboxylic acid is preferably selected from caprylic acid (C8), pelargonic acid (C9), capric acid (C10), undecylic acid (C11), lauric acid (C12), tridecylic acid (C13), myristic acid (C14), pentadecylic acid (C15), palmitic acid (C16), margaric acid (C17), stearic acid (C18), nonadecylic acid (C19), arachidic acid (C20), heneicosylic acid (C21), behenic acid (C22), tricosylic acid (C23), lignoceric acid (C24), pentacosylic acid (C25), cerotic acid (C26), and mixtures thereof.

Preferably, the metal salt of a carboxylic acid comprises zinc stearate, silver stearate, palladium stearate, zinc palmitate, silver palmitate, palladium palmitate, and mixtures thereof.

The metal salt of a carboxylic acid is present in the engine oil formulations of this disclosure in an amount of from about 0.01 weight percent to about 5 weight percent, based on the total weight of the formulated oil.

Low phosphorus engine oil formulations are included in this disclosure. For such formulations, the phosphorus content is typically less than about 0.12 weight percent preferably less than about 0.10 weight percent and most preferably less than about 0.085 weight percent.

A metal alkylthiophosphate and more particularly a metal dialkyl dithio phosphate in which the metal constituent is zinc, or zinc dialkyl dithio phosphate (ZDDP) can be a useful component of the lubricating oils of this disclosure. ZDDP can be derived from primary alcohols, secondary alcohols or mixtures thereof. ZDDP compounds generally are of the formula

$$Zn[SP(S)(OR^1)(OR^2)]_2$$

where $R^1$ and $R^2$ are $C_1$-$C_{18}$ alkyl groups, preferably $C_2$-$C_{12}$ alkyl groups. These alkyl groups may be straight chain or branched. Alcohols used in the ZDDP can be 2-propanol, butanol, secondary butanol, pentanols, hexanols such as 4-methyl-2-pentanol, n-hexanol, n-octanol, 2-ethyl hexanol, alkylated phenols, and the like. Mixtures of secondary alcohols or of primary and secondary alcohol can be preferred. Alkyl aryl groups may also be used.

Preferable zinc dithiophosphates which are commercially available include secondary zinc dithiophosphates such as those available from for example, The Lubrizol Corporation under the trade designations "LZ 677A", "LZ 1095" and "LZ 1371", from for example Chevron Oronite under the trade designation "OLOA 262" and from for example Afton Chemical under the trade designation "HITEC 7169".

The ZDDP is typically used in amounts of from about 0.4 weight percent to about 1.2 weight percent, preferably from about 0.5 weight percent to about 1.0 weight percent, and more preferably from about 0.6 weight percent to about 0.8 weight percent, based on the total weight of the lubricating oil, although more or less can often be used advantageously. Preferably, the ZDDP is a secondary ZDDP and present in an amount of from about 0.6 to 1.0 weight percent of the total weight of the lubricating oil.

Low phosphorus engine oil formulations are included in this disclosure. For such formulations, the phosphorus content is typically less than about 0.12 weight percent preferably less than about 0.10 weight percent and most preferably less than about 0.085 weight percent.

Dispersants

During engine operation, oil-insoluble oxidation byproducts are produced. Dispersants help keep these byproducts in solution, thus diminishing their deposition on metal surfaces. Dispersants used in the formulation of the lubricating oil may be ashless or ash-forming in nature. Preferably, the dispersant is ashless. So called ashless dispersants are organic materials that form substantially no ash upon combustion. For example, non-metal-containing or borated metal-free dispersants are considered ashless. In contrast, metal-containing detergents discussed above form ash upon combustion.

Suitable dispersants typically contain a polar group attached to a relatively high molecular weight hydrocarbon chain. The polar group typically contains at least one element of nitrogen, oxygen, or phosphorus. Typical hydrocarbon chains contain 50 to 400 carbon atoms.

A particularly useful class of dispersants are the (poly)alkenylsuccinic derivatives, typically produced by the reaction of a long chain hydrocarbyl substituted succinic compound, usually a hydrocarbyl substituted succinic anhydride, with a polyhydroxy or polyamino compound. The long chain hydrocarbyl group constituting the oleophilic portion of the molecule which confers solubility in the oil, is normally a polyisobutylene group. Many examples of this type of dispersant are well known commercially and in the literature. Exemplary U.S. patents describing such dispersants are U.S. Patent Nos. 3,172,892; 3,2145,707; 3,219,666; 3,316,177; 3,341,542; 3,444,170; 3,454,607; 3,541,012; 3,630,904; 3,632,511; 3,787,374 and 4,234,435. Other types of dispersant are described in U.S. Patent Nos. 3,036,003; 3,200,107; 3,254,025; 3,275,554; 3,438,757; 3,454,555; 3,565,804; 3,413,347; 3,697,574; 3,725,277; 3,725,480; 3,726,882; 4,454,059; 3,329,658; 3,449,250; 3,519,565; 3,666,730; 3,687,849; 3,702,300; 4,100,082; 5,705,458. A further description of dispersants may be found, for example, in European Patent Application No. 471 071, to which reference is made for this purpose.

Hydrocarbyl-substituted succinic acid and hydrocarbyl-substituted succinic anhydride derivatives are useful dispersants. In particular, succinimide, succinate esters, or succinate ester amides prepared by the reaction of a hydrocarbon-substituted succinic acid compound preferably having at least 50 carbon atoms in the hydrocarbon substituent, with at least one equivalent of an alkylene amine are particularly useful.

Succinimides are formed by the condensation reaction between hydrocarbyl substituted succinic anhydrides and amines. Molar ratios can vary depending on the polyamine. For example, the molar ratio of hydrocarbyl substituted succinic anhydride to TEPA can vary from about 1:1 to about 5:1. Representative examples are shown in U.S. Patent Nos. 3,087,936; 3,172,892; 3,219,666; 3,272,746; 3,322,670; and 3,652,616, 3,948,800; and Canada Patent No. 1,094,044.

Succinate esters are formed by the condensation reaction between hydrocarbyl substituted succinic anhydrides and alcohols or polyols. Molar ratios can vary depending on the alcohol or polyol used. For example, the condensation product of a hydrocarbyl substituted succinic anhydride and pentaerythritol is a useful dispersant.

Succinate ester amides are formed by condensation reaction between hydrocarbyl substituted succinic anhydrides and alkanol amines. For example, suitable alkanol amines include ethoxylated polyalkylpolyamines, propoxylated polyalkylpolyamines and polyalkenylpolyamines such as polyethylene polyamines. One example is propoxylated hexamethylenediamine. Representative examples are shown in U.S. Patent No. 4,426,305.

The molecular weight of the hydrocarbyl substituted succinic anhydrides used in the preceding paragraphs will typically range between 800 and 2,500 or more. The above products can be post-reacted with various reagents such as sulfur, oxygen, formaldehyde, carboxylic acids such as oleic acid. The above products can also be post reacted with boron compounds such as boric acid, borate esters or highly borated dispersants, to form borated dispersants generally having from about 0.1 to about 5 moles of boron per mole of dispersant reaction product.

Mannich base dispersants are made from the reaction of alkylphenols, formaldehyde, and amines. See U.S. Patent No. 4,767,551. Process aids and catalysts, such as oleic acid and sulfonic acids, can also be part of the reaction mixture. Molecular weights of the alkylphenols range from 800 to 2,500. Representative examples are shown in U.S. Patent Nos. 3,697,574; 3,703,536; 3,704,308; 3,751,365; 3,756,953; 3,798,165; and 3,803,039.

Typical high molecular weight aliphatic acid modified Mannich condensation products useful in this disclosure can be prepared from high molecular weight alkyl-substituted hydroxyaromatics or $HNR_2$ group-containing reactants.

Hydrocarbyl substituted amine ashless dispersant additives are well known to one skilled in the art; see, for example, U.S. Patent Nos. 3,275,554; 3,438,757; 3,565,804; 3,755,433, 3,822,209, and 5,084,197.

Preferred dispersants include borated and non-borated succinimides, including those derivatives from mono-succinimides, bis-succinimides, and/or mixtures of mono- and bis-succinimides, wherein the hydrocarbyl succinimide is derived from a hydrocarbylene group such as polyisobutylene having a Mn of from about 500 to about 5000, or from about 1000 to about 3000, or about 1000 to about 2000, or a mixture of such hydrocarbylene groups, often with high terminal vinylic groups. Other preferred dispersants include succinic acid-esters and amides, alkylphenol-polyamine-coupled Mannich adducts, their capped derivatives, and other related components.

Polymethacrylate or polyacrylate derivatives are another class of dispersants. These dispersants are typically prepared by reacting a nitrogen containing monomer and a methacrylic or acrylic acid esters containing 5 -25 carbon atoms in the ester group. Representative examples are shown in U.S. Patent Nos. 2, 100, 993, and 6,323,164. Polymethacrylate and polyacrylate dispersants are normally used as multifunctional viscosity modifiers. The lower molecular weight versions can be used as lubricant dispersants or fuel detergents.

Illustrative preferred dispersants useful in this disclosure include those derived from polyalkenyl-substituted mono- or dicarboxylic acid, anhydride or ester, which dispersant has a polyalkenyl moiety with a number average molecular weight of at least 900 and from greater than 1.3 to 1.7, preferably from greater than 1.3 to 1.6, most preferably

from greater than 1.3 to 1.5, functional groups (mono- or dicarboxylic acid producing moieties) per polyalkenyl moiety (a medium functionality dispersant). Functionality (F) can be determined according to the following formula:

$$F = (SAP \times M_n)/((112{,}200 \times A.I.)-(SAP \times 98))$$

wherein SAP is the saponification number (i.e., the number of milligrams of KOH consumed in the complete neutralization of the acid groups in one gram of the succinic-containing reaction product, as determined according to ASTM D94); $M_n$ is the number average molecular weight of the starting olefin polymer; and A.I. is the percent active ingredient of the succinic-containing reaction product (the remainder being unreacted olefin polymer, succinic anhydride and diluent).

The polyalkenyl moiety of the dispersant may have a number average molecular weight of at least 900, suitably at least 1500, preferably between 1800 and 3000, such as between 2000 and 2800, more preferably from about 2100 to 2500, and most preferably from about 2200 to about 2400. The molecular weight of a dispersant is generally expressed in terms of the molecular weight of the polyalkenyl moiety. This is because the precise molecular weight range of the dispersant depends on numerous parameters including the type of polymer used to derive the dispersant, the number of functional groups, and the type of nucleophilic group employed.

Polymer molecular weight, specifically $M_n$, can be determined by various known techniques. One convenient method is gel permeation chromatography (GPC), which additionally provides molecular weight distribution information (see W. W. Yau, J. J. Kirkland and D. D. Bly, "Modern Size Exclusion Liquid Chromatography", John Wiley and Sons, New York, 1979). Another useful method for determining molecular weight, particularly for lower molecular weight polymers, is vapor pressure osmometry (e.g., ASTM D3592).

The polyalkenyl moiety in a dispersant preferably has a narrow molecular weight distribution (MWD), also referred to as polydispersity, as determined by the ratio of weight average molecular weight ($M_w$) to number average molecular weight ($M_n$). Polymers having a $M_w/M_n$ of less than 2.2, preferably less than 2.0, are most desirable. Suitable polymers have a polydispersity of from about 1.5 to 2.1, preferably from about 1.6 to about 1.8.

Suitable polyalkenes employed in the formation of the dispersants include homopolymers, interpolymers or lower molecular weight hydrocarbons. One family of such polymers comprise polymers of ethylene and/or at least one $C_3$ to $C_2$ alpha-olefin having the formula $H_2C = CHR^1$ wherein $R^1$ is a straight or branched chain alkyl radical comprising 1 to 26 carbon atoms and wherein the polymer contains carbon-to-carbon unsaturation, and a high degree of terminal ethenylidene unsaturation. Preferably, such polymers comprise interpolymers of ethylene and at least one alpha-olefin of the above formula, wherein $R^1$ is alkyl of from 1 to 18 carbon atoms, and more preferably is alkyl of from 1 to 8 carbon atoms, and more preferably still of from 1 to 2 carbon atoms.

Another useful class of polymers is polymers prepared by cationic polymerization of monomers such as isobutene and styrene. Common polymers from this class include polyisobutenes obtained by polymerization of a $C_4$ refinery stream having a butene content of 35 to 75% by wt., and an isobutene content of 30 to 60% by wt. A preferred source of monomer for making poly-n-butenes is petroleum feedstreams such as Raffinate II. These feedstocks are disclosed in the art such as in U.S. Pat. No. 4,952,739. A preferred embodiment utilizes polyisobutylene prepared from a pure isobutylene stream or a Raffinate I stream to prepare reactive isobutylene polymers with terminal vinylidene olefins. Polyisobutene polymers that may be employed are generally based on a polymer chain of from 1500 to 3000.

The dispersant(s) are preferably non-polymeric (e.g., mono- or bis-succinimides). Such dispersants can be prepared by conventional processes such as disclosed in U.S. Patent Application Publication No. 2008/0020950.

The dispersant(s) can be borated by conventional means, as generally disclosed in U.S. Patent Nos. 3,087,936, 3,254,025 and 5,430,105.

Such dispersants may be used in an amount of about 0.01 to 20 weight percent or 0.01 to 10 weight percent, preferably about 0.5 to 8 weight percent, or more preferably 0.5 to 4 weight percent. Or such dispersants may be used in an amount of about 2 to 12 weight percent, preferably about 4 to 10 weight percent, or more preferably 6 to 9 weight percent. On an active ingredient basis, such additives may be used in an amount of about 0.06 to 14 weight percent, preferably about 0.3 to 6 weight percent. The hydrocarbon portion of the dispersant atoms can range from $C_{60}$ to $C_{1000}$, or from $C_{70}$ to $C_{300}$, or from $C_{70}$ to $C_{200}$. These dispersants may contain both neutral and basic nitrogen, and mixtures of both. Dispersants can be end-capped by borates and/or cyclic carbonates.

As used herein, the dispersant concentrations are given on an "as delivered" basis. Typically, the active dispersant is delivered with a process oil. The "as delivered" dispersant typically contains from about 20 weight percent to about 80 weight percent, or from about 40 weight percent to about 60 weight percent, of active dispersant in the "as delivered" dispersant product.

Viscosity Modifiers

Viscosity modifiers (also known as viscosity index improvers (VI improvers), and viscosity improvers) can be

included in the lubricant compositions of this disclosure.

Viscosity modifiers provide lubricants with high and low temperature operability. These additives impart shear stability at elevated temperatures and acceptable viscosity at low temperatures.

Suitable viscosity modifiers include high molecular weight hydrocarbons, polyesters and viscosity modifier dispersants that function as both a viscosity modifier and a dispersant. Typical molecular weights of these polymers are between about 10,000 to 1,500,000, more typically about 20,000 to 1,200,000, and even more typically between about 50,000 and 1,000,000.

Examples of suitable viscosity modifiers are linear or star-shaped polymers and copolymers of methacrylate, butadiene, olefins, or alkylated styrenes. Polyisobutylene is a commonly used viscosity modifier. Another suitable viscosity modifier is polymethacrylate (copolymers of various chain length alkyl methacrylates, for example), some formulations of which also serve as pour point depressants. Other suitable viscosity modifiers include copolymers of ethylene and propylene, hydrogenated block copolymers of styrene and isoprene, and polyacrylates (copolymers of various chain length acrylates, for example). Specific examples include styrene-isoprene or styrene-butadiene based polymers of 50,000 to 200,000 molecular weight.

Olefin copolymers are commercially available from Chevron Oronite Company LLC under the trade designation "PARATONE®" (such as "PARATONE® 8921" and "PARATONE® 8941"); from Afton Chemical Corporation under the trade designation "HiTEC®" (such as "HiTEC® 5850B"; and from The Lubrizol Corporation under the trade designation "Lubrizol® 7067C". Hydrogenated polyisoprene star polymers are commercially available from Infineum International Limited, e.g., under the trade designation "SV200" and "SV600". Hydrogenated diene-styrene block copolymers are commercially available from Infineum International Limited, e.g., under the trade designation "SV 50".

The polymethacrylate or polyacrylate polymers can be linear polymers which are available from Evnoik Industries under the trade designation "Viscoplex®" (e.g., Viscoplex 6-954) or star polymers which are available from Lubrizol Corporation under the trade designation Asteric™ (e.g., Lubrizol 87708 and Lubrizol 87725).

Illustrative vinyl aromatic-containing polymers useful in this disclosure may be derived predominantly from vinyl aromatic hydrocarbon monomer. Illustrative vinyl aromatic-containing copolymers useful in this disclosure may be represented by the following general formula:

A-B

wherein A is a polymeric block derived predominantly from vinyl aromatic hydrocarbon monomer, and B is a polymeric block derived predominantly from conjugated diene monomer.

In an embodiment of this disclosure, the viscosity modifiers may be used in an amount of less than about 2.0 weight percent, preferably less than about 1.0 weight percent, and more preferably less than about 0.5 weight percent, based on the total weight of the formulated oil or lubricating engine oil. Viscosity modifiers are typically added as concentrates, in large amounts of diluent oil.

As used herein, the viscosity modifier concentrations are given on an "as delivered" basis. Typically, the active polymer is delivered with a diluent oil. The "as delivered" viscosity modifier typically contains from 20 weight percent to 75 weight percent of an active polymer for polymethacrylate or polyacrylate polymers, or from 8 weight percent to 20 weight percent of an active polymer for olefin copolymers, hydrogenated polyisoprene star polymers, or hydrogenated diene-styrene block copolymers, in the "as delivered" polymer concentrate.

Antioxidants

Antioxidants retard the oxidative degradation of base oils during service. Such degradation may result in deposits on metal surfaces, the presence of sludge, or a viscosity increase in the lubricant. One skilled in the art knows a wide variety of oxidation inhibitors that are useful in lubricating oil compositions. See, Klamann in Lubricants and Related Products, op cite, and U.S. Patent Nos. 4,798,684 and 5,084,197, for example.

Useful antioxidants include hindered phenols. These phenolic antioxidants may be ashless (metal-free) phenolic compounds or neutral or basic metal salts of certain phenolic compounds. Typical phenolic antioxidant compounds are the hindered phenolics which are the ones which contain a sterically hindered hydroxyl group, and these include those derivatives of dihydroxy aryl compounds in which the hydroxyl groups are in the o- or p-position to each other. Typical phenolic antioxidants include the hindered phenols substituted with $C_6$+ alkyl groups and the alkylene coupled derivatives of these hindered phenols. Examples of phenolic materials of this type 2-t-butyl-4-heptyl phenol; 2-t-butyl-4-octyl phenol; 2-t-butyl-4-dodecyl phenol; 2,6-di-t-butyl-4-heptyl phenol; 2,6-di-t-butyl-4-dodecyl phenol; 2-methyl-6-t-butyl-4-heptyl phenol; and 2-methyl-6-t-butyl-4-dodecyl phenol. Other useful hindered mono-phenolic antioxidants may include for example hindered 2,6-di-alkyl-phenolic proprionic ester derivatives. Bis-phenolic antioxidants may also be advantageously used in combination with the instant disclosure. Examples of ortho-coupled phenols include: 2,2'-bis(4-heptyl-6-t-butyl-phenol); 2,2'-bis(4-octyl-6-t-butyl-phenol); and 2,2'-bis(4-dodecyl-6-t-butyl-phenol). Para-coupled bisphenols

include for example 4,4'-bis(2,6-di-t-butyl phenol) and 4,4'-methylene-bis(2,6-di-t-butyl phenol).

Effective amounts of one or more catalytic antioxidants may also be used. The catalytic antioxidants comprise an effective amount of a) one or more oil soluble polymetal organic compounds; and, effective amounts of b) one or more substituted N,N'-diaryl-o-phenylenediamine compounds or c) one or more hindered phenol compounds; or a combination of both b) and c). Catalytic antioxidants are more fully described in U.S. Patent No. 8, 048,833.

Non-phenolic oxidation inhibitors which may be used include aromatic amine antioxidants and these may be used either as such or in combination with phenolics. Typical examples of non-phenolic antioxidants include: alkylated and non-alkylated aromatic amines such as aromatic monoamines of the formula $R^8R^9R^{10}N$ where $R^8$ is an aliphatic, aromatic or substituted aromatic group, $R^9$ is an aromatic or a substituted aromatic group, and $R^{10}$ is H, alkyl, aryl or $R^{11}S(O)xR^{12}$ where $R^{11}$ is an alkylene, alkenylene, or aralkylene group, $R^{12}$ is a higher alkyl group, or an alkenyl, aryl, or alkaryl group, and x is 0, 1 or 2. The aliphatic group $R^8$ may contain from 1 to about 20 carbon atoms, and preferably contains from about 6 to 12 carbon atoms. The aliphatic group is a saturated aliphatic group. Preferably, both $R^8$ and $R^9$ are aromatic or substituted aromatic groups, and the aromatic group may be a fused ring aromatic group such as naphthyl. Aromatic groups $R^8$ and $R^9$ may be joined together with other groups such as S.

Typical aromatic amines antioxidants have alkyl substituent groups of at least about 6 carbon atoms. Examples of aliphatic groups include hexyl, heptyl, octyl, nonyl, and decyl. Generally, the aliphatic groups will not contain more than about 14 carbon atoms. The general types of amine antioxidants useful in the present compositions include diphenylamines, phenyl naphthylamines, phenothiazines, imidodibenzyls and diphenyl phenylene diamines. Mixtures of two or more aromatic amines are also useful. Polymeric amine antioxidants can also be used. Particular examples of aromatic amine antioxidants useful in the present disclosure include: p,p'-dioctyldiphenylamine; t-octylphenyl-alpha-naphthylamine; phenyl-alphanaphthylamine; and p-octylphenyl-alpha-naphthylamine.

Sulfurized alkyl phenols and alkali or alkaline earth metal salts thereof also are useful antioxidants.

Preferred antioxidants include hindered phenols, arylamines. These antioxidants may be used individually by type or in combination with one another. Such additives may be used in an amount of about 0.01 to 5 weight percent, preferably about 0.01 to 1.5 weight percent, more preferably zero to less than 1.5 weight percent, more preferably zero to less than 1 weight percent.

Pour Point Depressants (PPDs)

Conventional pour point depressants (also known as lube oil flow improvers) may be added to the compositions of the present disclosure if desired. These pour point depressant may be added to lubricating compositions of the present disclosure to lower the minimum temperature at which the fluid will flow or can be poured. Examples of suitable pour point depressants include polymethacrylates, polyacrylates, polyarylamides, condensation products of haloparaffin waxes and aromatic compounds, vinyl carboxylate polymers, and terpolymers of dialkylfumarates, vinyl esters of fatty acids and allyl vinyl ethers. U.S. Patent Nos. 1,815,022; 2,015,748; 2,191,498; 2,387,501; 2,655, 479; 2,666,746; 2,721,877; 2,721,878; and 3,250,715 describe useful pour point depressants and/or the preparation thereof. Such additives may be used in an amount of about 0.01 to 5 weight percent, preferably about 0.01 to 1.5 weight percent.

Seal Compatibility Agents

Seal compatibility agents help to swell elastomeric seals by causing a chemical reaction in the fluid or physical change in the elastomer. Suitable seal compatibility agents for lubricating oils include organic phosphates, aromatic esters, aromatic hydrocarbons, esters (butylbenzyl phthalate, for example), and polybutenyl succinic anhydride. Such additives may be used in an amount of about 0.01 to 3 weight percent, preferably about 0.01 to 2 weight percent.

Antifoam Agents

Anti-foam agents may advantageously be added to lubricant compositions. These agents retard the formation of stable foams. Silicones and organic polymers are typical anti-foam agents. For example, polysiloxanes, such as silicon oil or polydimethyl siloxane, provide antifoam properties. Anti-foam agents are commercially available and may be used in conventional minor amounts along with other additives such as demulsifiers; usually the amount of these additives combined is less than 1 weight percent and often less than 0.1 weight percent.

Inhibitors and Antirust Additives

Antirust additives (or corrosion inhibitors) are additives that protect lubricated metal surfaces against chemical attack by water or other contaminants. A wide variety of these are commercially available.

One type of antirust additive is a polar compound that wets the metal surface preferentially, protecting it with

a film of oil. Another type of antirust additive absorbs water by incorporating it in a water-in-oil emulsion so that only the oil touches the metal surface. Yet another type of antirust additive chemically adheres to the metal to produce a non-reactive surface. Examples of suitable additives include zinc dithiophosphates, metal phenolates, basic metal sulfonates, fatty acids and amines. Such additives may be used in an amount of about 0.01 to 5 weight percent, preferably about 0.01 to 1.5 weight percent.

Friction Modifiers

A friction modifier is any material or materials that can alter the coefficient of friction of a surface lubricated by any lubricant or fluid containing such material(s). Friction modifiers, also known as friction reducers, or lubricity agents or oiliness agents, and other such agents that change the ability of base oils, formulated lubricant compositions, or functional fluids, to modify the coefficient of friction of a lubricated surface may be effectively used in combination with the base oils or lubricant compositions of the present disclosure if desired. Friction modifiers that lower the coefficient of friction are particularly advantageous in combination with the base oils and lube compositions of this disclosure.

Illustrative friction modifiers may include, for example, organometallic compounds or materials, or mixtures thereof. Illustrative organometallic friction modifiers useful in the lubricating engine oil formulations of this disclosure include, for example, molybdenum amine, molybdenum diamine, an organotungstenate, a molybdenum dithiocarbamate, molybdenum dithiophosphates, molybdenum amine complexes, molybdenum carboxylates, and the like, and mixtures thereof. Similar tungsten based compounds may be preferable.

Other illustrative friction modifiers useful in the lubricating engine oil formulations of this disclosure include, for example, alkoxylated fatty acid esters, alkanolamides, polyol fatty acid esters, borated glycerol fatty acid esters, fatty alcohol ethers, and mixtures thereof.

Illustrative alkoxylated fatty acid esters include, for example, polyoxyethylene stearate, fatty acid polyglycol ester, and the like. These can include polyoxypropylene stearate, polyoxybutylene stearate, polyoxyethylene isosterate, polyoxypropylene isostearate, polyoxyethylene palmitate, and the like.

Illustrative alkanolamides include, for example, lauric acid diethylalkanolamide, palmic acid diethylalkanolamide, and the like. These can include oleic acid diethyalkanolamide, stearic acid diethylalkanolamide, oleic acid diethylalkanolamide, polyethoxylated hydrocarbylamides, polypropoxylated hydrocarbylamides, and the like.

Illustrative polyol fatty acid esters include, for example, glycerol mono-oleate, saturated mono-, di-, and tri-glyceride esters, glycerol monostearate, and the like. These can include polyol esters, hydroxyl-containing polyol esters, and the like.

Illustrative borated glycerol fatty acid esters include, for example, borated glycerol mono-oleate, borated saturated mono-, di-, and tri-glyceride esters, borated glycerol mono-sterate, and the like. In addition to glycerol polyols, these can include trimethylolpropane, pentaerythritol, sorbitan, and the like. These esters can be polyol monocarboxylate esters, polyol dicarboxylate esters, and on occasion polyoltricarboxylate esters. Preferred can be the glycerol mono-oleates, glycerol dioleates, glycerol trioleates, glycerol monostearates, glycerol distearates, and glycerol tristearates and the corresponding glycerol monopalmitates, glycerol dipalmitates, and glycerol tripalmitates, and the respective isostearates, linoleates, and the like. On occasion the glycerol esters can be preferred as well as mixtures containing any of these. Ethoxylated, propoxylated, butoxylated fatty acid esters of polyols, especially using glycerol as underlying polyol can be preferred.

Illustrative fatty alcohol ethers include, for example, stearyl ether, myristyl ether, and the like. Alcohols, including those that have carbon numbers from $C_3$ to $C_{50}$, can be ethoxylated, propoxylated, or butoxylated to form the corresponding fatty alkyl ethers. The underlying alcohol portion can preferably be stearyl, myristyl, $C_{11}$ - $C_{13}$ hydrocarbon, oleyl, isosteryl, and the like.

The lubricating oils of this disclosure exhibit desired properties, e.g., wear control, in the presence or absence of a friction modifier.

Useful concentrations of friction modifiers may range from 0.01 weight percent to 5 weight percent, or about 0.1 weight percent to about 2.5 weight percent, or about 0.1 weight percent to about 1.5 weight percent, or about 0.1 weight percent to about 1 weight percent. Concentrations of molybdenum-containing materials are often described in terms of Mo metal concentration. Advantageous concentrations of Mo may range from 25 ppm to 700 ppm or more, and often with a preferred range of 50-200 ppm. Friction modifiers of all types may be used alone or in mixtures with the materials of this disclosure. Often mixtures of two or more friction modifiers, or mixtures of friction modifier(s) with alternate surface active material(s), are also desirable.

When lubricating oil compositions contain one or more of the additives discussed above, the additive(s) are blended into the composition in an amount sufficient for it to perform its intended function. Typical amounts of such additives useful in the present disclosure are shown in Table 1 below.

It is noted that many of the additives are shipped from the additive manufacturer as a concentrate, containing one or more additives together, with a certain amount of base oil diluents. Accordingly, the weight amounts in the table

below, as well as other amounts mentioned herein, are directed to the amount of active ingredient (that is the non-diluent portion of the ingredient). The weight percent (wt%) indicated below is based on the total weight of the lubricating oil composition.

TABLE 1

Typical Amounts of Other Lubricating Oil Components

| Compound | Approximate wt% (Useful) | Approximate wt% (Preferred) |
|---|---|---|
| Dispersant | 0.1-20 | 0.1-8 |
| Detergent | 0.1-20 | 0.1-8 |
| Friction Modifier | 0.01-5 | 0.01-1.5 |
| Antioxidant | 0.1-5 | 0.1-1.5 |
| Pour Point Depressant (PPD) | 0.0-5 | 0.01-1.5 |
| Anti-foam Agent | 0.001-3 | 0.001-0.15 |
| Viscosity Modifier (solid polymer basis) | 0.1-2 | 0.1-1 |
| Antiwear | 0.2-3 | 0.5-1 |
| Inhibitor and Antirust | 0.01-5 | 0.01-1.5 |

The foregoing additives are all commercially available materials. These additives may be added independently but are usually precombined in packages which can be obtained from suppliers of lubricant oil additives. Additive packages with a variety of ingredients, proportions and characteristics are available and selection of the appropriate package will take the requisite use of the ultimate composition into account.

The following non-limiting examples are provided to illustrate the disclosure.

## EXAMPLES

Fig. 1 shows examples of "detect" and "no detect" from immunoassay interrogation of a lubricating oil in accordance with this disclosure.

Fig. 2 shows examples of a chemical bar code for determining product authenticity in accordance with this disclosure.

Fig. 3 shows an example of a chemical bar code for determining product authenticity (A-E corresponds to product authenticity) and identity (1-8 corresponds to product identity) in accordance with this disclosure. With the taggant array combined with a product ID array, this allows an end user to not only verify product authenticity, but also determine the product identity in an overt manner and prevent mislabeling errors.

When numerical lower limits and numerical upper limits are listed herein, ranges from any lower limit to any upper limit are contemplated. While the illustrative embodiments of the disclosure have been described with particularity, it will be understood that various other modifications will be apparent to and can be readily made by those skilled in the art. Accordingly, it is not intended that the scope of the claims appended hereto be limited to the examples and descriptions set forth herein.

## Claims

**1.** A method comprising:

associating a taggant with a product to produce a lubricating oil signature product; wherein the taggant, when associated with the signature product, is visually undetectable; and wherein the taggant comprises one or more amide compounds, wherein the one or more amide compounds are not proteins;
identifying the taggant in the signature product by an immunoassay specific for the taggant;
mapping the taggant of the signature product to a batch code of the signature product;
obtaining a lubricating oil test product to determine authenticity of the test product;
identifying the presence or absence of a taggant in the test product by an immunoassay specific for the taggant; and
comparing results of the immunoassay carried out on the test product with results of the immunoassay carried out on the signature product to determine authenticity of the test product, wherein the immunoassay is carried out using a test strip that is specific for the taggant.

**2.** A method comprising:

associating a taggant with a product to produce a lubricating oil signature product; wherein the taggant, when associated with the signature product, is visually undetectable; and wherein the taggant comprises one or more amide compounds, wherein the one or more amide compounds are not proteins;
identifying the taggant in the signature product by an immunoassay specific for the taggant;
mapping the taggant of the signature product to a product code of the signature product;
obtaining a lubricating oil test product to determine identification of the test product;
identifying the presence or absence of a taggant in the test product by an immunoassay specific for the taggant; and
comparing results of the immunoassay carried out on the test product with results of the immunoassay carried out on the signature product to determine identification of the test product, wherein the immunoassay is carried out using a test strip that is specific for the taggant.

**3.** The method of claims 1 and 2 wherein the taggant comprises a taggant array, and wherein the taggant array comprises two or more amide compounds.

**4.** The method of claims 1-3 further comprising mapping the taggant of the signature product to a batch code or a product code of the signature product through the use of a decoder key.

**5.** The method of claims 1-4 further comprising obtaining the mapped taggant of the signature product to the batch code or the product code of the signature product from a supplier website or database.

**6.** The method of claims 1-5 further comprising comparing the mapped taggant of the signature product to the batch code or the product code of the signature product with an immunoassay carried out on a purchased product to determine authenticity of the purchased product.

**7.** The method of claims 1-6 wherein the test strip is a coded test strip that can be read by a bar code reader.

**8.** The method of claims 1-7 wherein the test strip comprises a taggant and a product identification.

**9.** The method of claims 1-8 wherein the test strip is a lateral flow immunoassay.

**10.** The method of claims 1-9 wherein the taggant comprises one or more aliphatic amide compounds or one or more cyclic amide compounds.

**11.** The method of claims 1-10 wherein the aliphatic amide compounds are selected from pyridine-3-carboxylic acid amide, N-(1-cyano-1,2-dimethylpropyl)-2-(2,4-dichlorophenoxy)propanamide, 1-benzoyl-4-propionylpiperazine, (2S)-2-{[(2S)-2-aminobutanoyl] amino }propanoic acid, 3-[decyl(dimethyl)silyl]-N-[2-(4-methylphenyl)-1-phenylethyl]propanamide, N-allyl-4,5-dimethyl-2-(trimethylsilyl)-3-thiophenecarboxamide, benzyl (1S)-2-({2-[(2-amino-2-oxoethyl)amino]-2-oxoethyl}amino)-1-benzyl-2-oxoethylcarbamate, benzyl(1S,2S)-1-({[(1R)-2-amino-1-benzyl-2-oxoethyl]amino}carbonyl)-2-hydroxypropylcarbamate, N-cyclohexyl-N-methyl-4-[(2-oxo-1,2-dihydro-6-quinolinyl)oxy]butanamide, N-[2-(1H-indol-3-yl)ethyl]tetracosanamide, and N-[2-(1H-indol-3-yl)ethyl]heptadecanamide.

**12.** The method of claims 1-11 wherein the cyclic amide compounds are selected from (5S)-1-methyl-5-(3-pyridyl)pyrrolidin-2-one, 1,3-diethyl-2-thioxodihydro-4,6(1H,5H)-pyrimidinedione, N-[2-(1H-indol-3-yl)ethyl]heptadecanamide, 3-{2-[4-(6-fluoro-l,2-benzisoxazol-3-yl)-1-piperidinyl]ethyl}-2-methyl-6,7,8,9-tetrahydro-4H-pyrido[1,2-a]pyrimidin-4-one, biotinyl-N-hydroxy-succinimide, 4-ethyl-2-pyrrolidinone, 2-azaspiro[4.6]undecan-3-one, 2-azaspiro[4.4]nonan-3-one, ethyl 2-oxo-3-pyrrolidinecarboxylate, ethyl 5-oxo-3-pyrrolidinecarboxylate, N-(2-ethylhexyl)-5-norbornene-2,3-dicarboximide, 1-(3-aminophenyl)-2-pyrrolidinone, N-(2,6-dimethylphenyl)-2-(2-oxo-1-pyrrolidinyl)acetamide, (3S)-5-oxo-1-[(1S)-1-phenylethyl]-3-pyrrolidinecarboxylic acid, methyl 2-{[(2,5-dioxo-1-pyrrolidinyl)oxy]carbonyl}benzoate, 4-[3-(cyclopentyloxy)-4-methoxyphenyl]-2-pyrrolidinone, 1-{[(7-hydroxy-2-oxo-2H-chromen-3-yl)carbonyl]oxy}-2,5-pyrrolidinedione, 2-(1-hydroxyundecyl)-1-(4-nitroanilino)-6-phenyl-4a,7a-dihydro-1H-pyrrolo[3,4-b]pyridine-5,7(2H,6H)-dione, 1-methyl-2-piperidinone, 1,5-dimethyl-2-piperidinone, 1,4,4-trimethyl-2,6-piperidinedione, 4-methyl-1-undecyl-2-piperidinone, 4-methyl-1-decyl-2-piperidinone, 1-(1-adamantyl)-2-piperidinone, 3,3-(butane-1,4-diyl)bis(l,8,8-trimethyl-3-azabicyclo[3.2.1]octane-2,4-dione), and tert-butyl 2,4-dioxo-1-piperidinecarboxylate.

13. The method of claims 1-12 wherein the taggant is present in an amount of from 0.05 ppm to 20 ppm.

**Patentansprüche**

1. Verfahren, bei dem
ein Taggant mit einem Produkt assoziiert wird, um ein Schmierölsignaturprodukt zu produzieren, wobei der Taggant, wenn er mit dem Signaturprodukt assoziiert wird, visuell nicht erkennbar ist, und wobei der Taggant eine oder mehrere Amidverbindungen umfasst, wobei die eine oder mehreren Amidverbindungen keine Proteine sind,
der Taggant in dem Signaturprodukt durch einen für den Taggant spezifischen Immunoassay identifiziert wird,
der Taggant des Signaturprodukts auf einen Chargencode des Signaturprodukts gemappt wird,
ein Schmieröltestprodukt erhalten wird, um die Authentizität des Testprodukts zu ermitteln,
die Anwesenheit oder Abwesenheit eines Taggants in dem Testprodukt durch einen für den Taggant spezifischen Immunoassay identifiziert wird, und
Ergebnisse des Immunoassays, der mit dem Testprodukt durchgeführt wird, mit Ergebnissen des Immunoassays verglichen wird, der mit dem Signaturprodukt durchgeführt wird, um die Authentizität des Testprodukts zu ermitteln, wobei der Immunoassay unter Verwendung eines Teststreifens durchgeführt wird, der für den Taggant spezifisch ist.

2. Verfahren, bei dem
ein Taggant mit einem Produkt assoziiert wird, um ein Schmierölsignaturprodukt zu produzieren, wobei der Taggant, wenn er mit dem Signaturprodukt assoziiert wird, visuell nicht erkennbar ist, und wobei der Taggant eine oder mehrere Amidverbindungen umfasst, wobei die eine oder mehreren Amidverbindungen keine Proteine sind,
der Taggant in dem Signaturprodukt durch einen für den Taggant spezifischen Immunoassay identifiziert wird,
der Taggant des Signaturprodukts auf einen Produktcode des Signaturprodukts gemappt wird,
ein Schmieröltestprodukt erhalten wird, um die Identifikation des Testprodukts zu ermitteln,
die Anwesenheit oder Abwesenheit eines Taggants in dem Testprodukt durch einen für den Taggant spezifischen Immunoassay identifiziert wird, und
Ergebnisse des Immunoassays, der mit dem Testprodukt durchgeführt wird, mit Ergebnissen des Immunoassays verglichen wird, der mit dem Signaturprodukt durchgeführt wird, um die Identifikation des Testprodukts zu ermitteln, wobei der Immunoassay unter Verwendung eines Teststreifens durchgeführt wird, der für den Taggant spezifisch ist.

3. Verfahren nach den Ansprüchen 1 und 2, bei dem der Taggant eine Taggant-Gruppierung umfasst, und wobei die Taggant-Gruppierung zwei oder mehr Amidverbindungen umfasst.

4. Verfahren nach den Ansprüchen 1 bis 3, das des Weiteren Mappen des Taggants des Signaturprodukts auf einen Chargencode oder einen Produktcode des Signaturprodukts durch die Verwendung eines Dekoderschlüssels umfasst.

5. Verfahren nach den Ansprüchen 1 bis 4, das des Weiteren Erhalten des auf den Chargencode oder den Produktcode des Signaturprodukts gemappten Taggants des Signaturprodukts von einer Website eines Lieferanten oder einer Datenbank umfasst.

6. Verfahren nach den Ansprüchen 1 bis 5, das des Weiteren Vergleichen des gemappten Taggants des Signaturprodukts mit dem Chargencode oder dem Produktcode des Signaturprodukts mit einem Immunoassay umfasst, der mit einem gekauften Produkt durchgeführt wird, um die Authentizität des gekauften Produkts zu ermitteln.

7. Verfahren nach den Ansprüchen 1 bis 6, bei dem der Teststreifen ein kodierter Teststreifen ist, der durch ein Barcodelesegerät gelesen werden kann.

8. Verfahren nach den Ansprüchen 1 bis 7, bei dem der Teststreifen einen Taggant und eine Produktidentifikation umfasst.

9. Verfahren nach den Ansprüchen 1 bis 8, bei dem der Teststreifen ein Lateral-Flow-Immunoassay (seitlicher Flusstest) ist.

10. Verfahren nach den Ansprüchen 1 bis 9, bei dem der Taggant eine oder mehrere aliphatische Amidverbindungen oder eine oder mehrere cyclische Amidverbindungen umfasst.

**11.** Verfahren nach den Ansprüchen 1 bis 10, bei dem die aliphatischen Amidverbindungen ausgewählt sind aus Pyridin-3-carbonsäureamid, N-(1-Cyano-1,2-dimethylpropyl)-2-(2,4-dichlorphenoxy)propanamid, 1-Benzoyl-4-propionylpiperazin, (2S)-2-{[(2S)-2-Aminobutanoyl]amino}propansäure, 3-[Decyl-(dimethyl)silyl]-N-[2-(4-methylphenyl)-1-phenylethyl]propanamid, N-Allyl-4,5-dimethyl-2-(trimethylsilyl)-3-thiophencarboxamid, Benzyl(1S)-2-({2-[(2-amino-2-oxoethyl)amino]-2-oxoethyl}amino)-1-benzyl-2-oxoethylcarbamat, Benzyl-(1S,2S)-1-({ [(1R)-2-amino-1-benzyl-2-oxoethyl]amino}carbonyl)-2-hydroxypropylcarbamat, N-Cyclohexyl-N-methyl-4-[(2-oxo-1,2-dihydro-6-chinolinyl)oxy]butanamid, N-[2-(1H-Indol-3-yl)ethyl]tetracosanamid und N-[2-(1H-Indol-3-yl)ethyl]heptadecanamid.

**12.** Verfahren nach den Ansprüchen 1 bis 11, bei dem die cyclischen Amidverbindungen ausgewählt sind aus (5S)-1-Methyl-5-(3-pyridyl)pyrrolidin-2-on, 1,3-Diethyl-2-thioxodihydro-4,6(1H,5H)-pyrimidindion, N-[2-(1H-Indol-3-yl)ethyl]-heptadecanamid, 3-{2-[4-(6-Fluor-1,2-benzisoxazol-3-yl)-1-piperidinyl]ethyl}-2-methyl-6,7,8,9-tetrahydro-4H-pyrido[1,2-a]pyrimidin-4-on, Biotinyl-N-hydroxysuccinimid, 4-Ethyl-2-pyrrolidinon, 2-Azaspiro[4.6]undecan-3-on, 2-Azaspiro[4.4]nonan-3-on, Ethyl-2-oxo-3-pyrrolidincarboxylat, Ethyl-5-oxo-3-pyrrolidincarboxylat, N-(2-Ethylhexyl)-5-norbornen-2,3-dicarboximid, 1-(3-Aminophenyl)-2-pyrrolidinon, N-(2,6-Dimethylphenyl)-2-(2-oxo-1-pyrrolidinyl)acetamid, (3S)-5-Oxo-1-[(1S)-1-phenylethyl]-3-pyrrolidincarbonsäure, Methyl-2-{[(2,5-dioxo-1-pyrrolidinyl)oxy]carbonyl}benzoat, 4-[3-(Cyclopentyloxy)-4-methoxyphenyl]-2-pyrrolidinon, 1-{[(7-Hydroxy-2-oxo-2H-chromen-3-yl)carbonyl]oxy}-2,5-pyrrolidindion, 2-(1-Hydroxyundecyl)-1-(4-nitroanilino)-6-phenyl-4a,7a-dihydro-1H-pyrrolo[3,4-b]pyridin-5,7(2H,6H)-dion, 1-Methyl-2-piperidinon, 1,5-Dimethyl-2-piperidinon, 1,4,4-Trimethyl-2,6-piperidindion, 4-Methyl-1-undecyl-2-piperidinon, 4-Methyl-1-decyl-2-piperidinon, 1-(1-Adamantyl)-2-piperidinon, 3,3-(Butan-1,4-diyl)bis(1,8,8-trimethyl-3-azabicyclo[3.2.1]octan-2,4-dion) und tert-Butyl-2,4-dioxo-1-piperidincarboxylat.

**13.** Verfahren nach den Ansprüchen 1 bis 12, bei dem der Taggant in einer Menge von 0,05 ppm bis 20 ppm vorhanden ist.

## Revendications

**1.** Procédé comprenant :

l'association d'un marqueur avec un produit pour produire un produit de signature d'huile lubrifiante ; le marqueur, lorsqu'il est associé au produit de signature, étant indétectable visuellement ; et le marqueur comprenant un ou plusieurs composés d'amide, le ou les composés d'amide n'étant pas des protéines ;
l'identification du marqueur dans le produit de signature par un dosage immunologique spécifique du marqueur ;
la mise en correspondance du marqueur du produit de signature avec un code de lot du produit de signature ;
l'obtention d'un produit d'essai d'huile lubrifiante pour déterminer l'authenticité du produit d'essai ;
l'identification de la présence ou l'absence d'un marqueur dans le produit d'essai par un dosage immunologique spécifique du marqueur ; et
la comparaison de résultats du dosage immunologique réalisé sur le produit d'essai avec des résultats du dosage immunologique réalisé sur le produit de signature pour déterminer l'authenticité du produit d'essai, le dosage immunologique étant réalisé au moyen d'une bandelette réactive qui est spécifique du marqueur.

**2.** Procédé comprenant :

l'association d'un marqueur avec un produit pour produire un produit de signature d'huile lubrifiante ; le marqueur, lorsqu'il est associé au produit de signature, étant indétectable visuellement ; et le marqueur comprenant un ou plusieurs composés d'amide, le ou les composés d'amide n'étant pas des protéines ;
l'identification du marqueur dans le produit de signature par un dosage immunologique spécifique du marqueur ;
la mise en correspondance du marqueur du produit de signature avec un code de produit du produit de signature ;
l'obtention d'un produit d'essai d'huile lubrifiante pour déterminer l'identification du produit d'essai ;
l'identification de la présence ou l'absence d'un marqueur dans le produit d'essai par un dosage immunologique spécifique du marqueur ; et
la comparaison de résultats du dosage immunologique réalisé sur le produit d'essai avec des résultats du dosage immunologique réalisé sur le produit de signature pour déterminer l'identification du produit d'essai, le dosage immunologique étant réalisé au moyen d'une bandelette réactive qui est spécifique du marqueur.

**3.** Procédé des revendications 1 et 2 dans lequel le marqueur comprend un réseau de marqueurs, et dans lequel le réseau de marqueurs comprend au moins deux composés d'amide.

**4.** Procédé des revendications 1 à 3 comprenant en outre la mise en correspondance du marqueur du produit de

signature avec un code de lot ou un code de produit du produit de signature par l'utilisation d'une clé de décodage.

5. Procédé des revendications 1 à 4 comprenant en outre l'obtention du marqueur du produit de signature mis en correspondance avec le code de lot ou le code de produit du produit de signature à partir d'un site Web ou d'une base de données de fournisseur.

6. Procédé des revendications 1 à 5 comprenant en outre la comparaison du marqueur du produit de signature mis en correspondance avec le code de lot ou le code de produit du produit de signature avec un dosage immunologique réalisé sur un produit acheté pour déterminer l'authenticité du produit acheté.

7. Procédé des revendications 1 à 6 dans lequel la bandelette réactive est une bandelette réactive codée qui peut être lue par un lecteur de codes à barres.

8. Procédé des revendications 1 à 7 dans lequel la bandelette réactive comprend un marqueur et une identification de produit.

9. Procédé des revendications 1 à 8 dans lequel la bandelette réactive est un dosage immunologique à flux latéral.

10. Procédé des revendications 1 à 9 dans lequel le marqueur comprend un ou plusieurs composés d'amide aliphatique ou un ou plusieurs composés d'amide cyclique.

11. Procédé des revendications 1 à 10 dans lequel les composés d'amide aliphatique sont choisis parmi l'amide d'acide pyridine-3-carboxylique, le N-(1-cyano-1,2-diméthylpropyl)-2-(2,4-dichlorophénoxy)propanamide, la 1-benzoyl-4-propionylpipérazine, l'acide (2S)-2-{[(2S)-2-aminobutanoyl]amino}propanoïque, le 3-[décyl-(diméthyl)silyl]-N-[2-(4-méthylphényl)-1-phényléthyl]-propanamide, le N-allyl-4,5-diméthyl-2-(triméthylsilyl)-3-thiophènecarboxamide, le (1S)-2-({2-[(2-amino-2-oxoéthyl)amino]-2-oxoéthyl}amino)-1-benzyl-2-oxoéthylcarbamate de benzyle, le (1S,2S)-1-({[(1R)-2-amino-1-benzyl-2-oxoéthyl]amino}carbonyl)-2-hydroxypropylcarbamate de benzyle, le N-cyclohexyl-N-méthyl-4-[(2-oxo-1,2-dihydro-6-quinolinyl)oxy]butanamide, le N-[2-(1H-indol-3-yl)éthyl]tétracosanamide, et le N-[2-(1H-indol-3-yl)éthyl]heptadécanamide.

12. Procédé des revendications 1 à 11 dans lequel les composés d'amide cyclique sont choisis parmi la (5S)-1-méthyl-5-(3-pyridyl)pyrrolidin-2-one, la 1,3-diéthyl-2-thioxodihydro-4,6(1H,5H)-pyrimidinedione, le N-[2-(1H-indol-3-yl)éthyl]heptadécanamide, la 3-{2-[4-(6-fluoro-1,2-benzisoxazol-3-yl)-1-pipéridinyl]éthyl}-2-méthyl-6,7,8,9-tétrahydro-4H-pyrido[1,2-a]pyrimidin-4-one, le biotinyl-N-hydroxysuccinimide, la 4-éthyl-2-pyrrolidinone, la 2-azaspiro[4.6]undécan-3-one, la 2-azaspiro[4.4]nonan-3-one, le 2-oxo-3-pyrrolidine-carboxylate d'éthyle, le 5-oxo-3-pyrrolidinecarboxylate d'éthyle, le N-(2-éthylhexyl)-5-norbornène-2,3-dicarboximide, la 1-(3-aminophényl)-2-pyrrolidinone, le N-(2,6-diméthylphényl)-2-(2-oxo-1-pyrrolidinyl)acétamide, l'acide (3S)-5-oxo-1-[(1S)-1-phényléthyl]-3-pyrrolidine-carboxylique, le 2-{[(2,5-dioxo-1-pyrrolidinyl)oxy]-carbonyl}benzoate de méthyle, la 4-[3-(cyclopentyloxy)-4-méthoxyphényl]-2-pyrrolidinone, la 1-{[(7-hydroxy-2-oxo-2H-chromén-3-yl)carbonyl]oxy}-2,5-pyrrolidinedione, la 2-(1-hydroxyundécyl)-1-(4-nitroanilino)-6-phényl-4a,7a-dihydro-1H-pyrrolo[3,4-b]pyridine-5,7(2H,6H)-dione, la 1-méthyl-2-pipéridinone, la 1,5-diméthyl-2-pipéridinone, la 1,4,4-triméthyl-2,6-pipéridinedione, la 4-méthyl-1-undécyl-2-pipéridinone, la 4-méthyl-1-décyl-2-pipéridinone, la 1-(1-adamantyl)-2-pipéridinone, la 3,3-(butane-1,4-diyl)bis(1,8,8-triméthyl-3-azabicyclo[3.2.1]octane-2,4-dione), et le 2,4-dioxo-1-pipéridinecarboxylate de tert-butyle.

13. Procédé des revendications 1 à 12 dans lequel le marqueur est présent dans une quantité de 0,05 ppm à 20 ppm.

Fig. 1

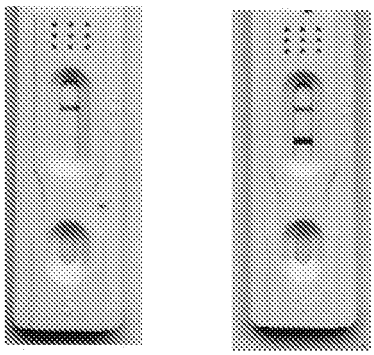

**Detect**

**(Authentic)**

**No Detect**

**(Counterfeit)**

Fig. 2

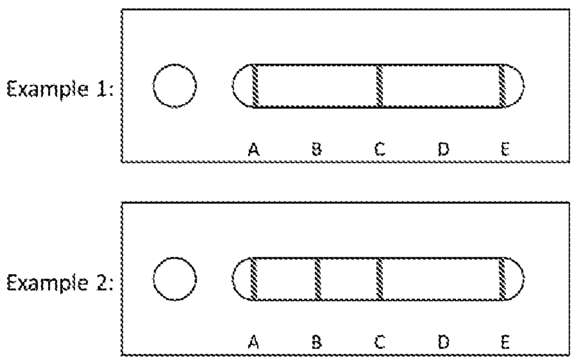

Fig. 3

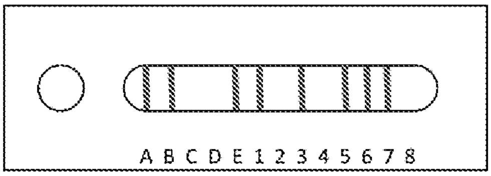

Fig. 4

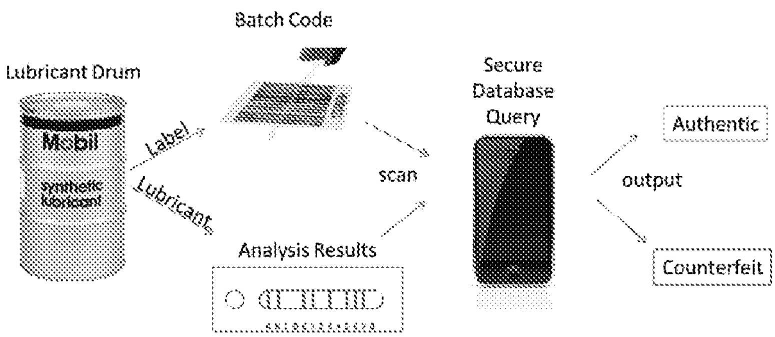
Batch Code
Lubricant Drum
Mobil
synthetic lubricant
Label
Lubricant
scan
Secure
Database
Query
Analysis Results
output
Authentic
Counterfeit

Fig. 5

| Tag Molecule | Chemical Category | Utility in Lubricant Anticounterfeiting |
|---|---|---|
| 1,3,5-Triazine-2,4,6-triamine | Nitrogen-Containing Small Molecule | Fail |
| Peanut Allergen Protein | Protein | Fail |
| Casein | Protein | Fail |
| Whey Protein | Protein | Fail |
| (5S)-1-methyl-5-(3-pyridyl)pyrrolidin-2-one | Nitrogen-Containing Small Molecule - Cyclic Amide | Success |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 0327163 A
- EP 0513604 A
- US 5984983 A
- US 4956122 A
- US 4827064 A
- US 4827073 A
- US 4149178 A
- US 3382291 A
- US 3742082 A
- US 3769363 A
- US 3876720 A
- US 4239930 A
- US 4367352 A
- US 4413156 A
- US 4434408 A
- US 4910355 A
- US 5068487 A
- US 4218330 A
- US 5075269 A
- US 2817693 A
- US 4975177 A
- US 4921594 A
- US 4897178 A
- GB 1429494 A
- GB 1350257 A
- GB 1440230 A
- GB 1390359 A
- EP 464546 A
- EP 464547 A
- US 4594172 A
- US 4943672 A
- US 6080301 A
- US 6090989 A
- US 6165949 A
- US 7704930 B
- US 3595791 A
- US 6034039 A
- US 3172892 A
- US 32145707 B
- US 3219666 A
- US 3316177 A
- US 3341542 A
- US 3444170 A
- US 3454607 A
- US 3541012 A
- US 3630904 A
- US 3632511 A
- US 3787374 A
- US 4234435 A
- US 3036003 A
- US 3200107 A
- US 3254025 A
- US 3275554 A
- US 3438757 A
- US 3454555 A
- US 3565804 A
- US 3413347 A
- US 3697574 A
- US 3725277 A
- US 3725480 A
- US 3726882 A
- US 4454059 A
- US 3329658 A
- US 3449250 A
- US 3519565 A
- US 3666730 A
- US 3687849 A
- US 3702300 A
- US 4100082 A
- US 5705458 A
- EP 471071 A
- US 3087936 A
- US 3272746 A
- US 3322670 A
- US 3652616 A
- US 3948800 A
- CA 1094044
- US 4426305 A
- US 4767551 A
- US 3703536 A
- US 3704308 A
- US 3751365 A
- US 3756953 A
- US 3798165 A
- US 3803039 A
- US 3755433 A
- US 3822209 A
- US 5084197 A
- US 2100993 A
- US 6323164 B
- US 4952739 A
- US 20080020950
- US 5430105 A
- US 4798684 A
- US 8048833 B
- US 1815022 A
- US 2015748 A
- US 2191498 A

- US 2387501 A
- US 2655 A
- US 479 A
- US 2666746 A
- US 2721877 A
- US 2721878 A
- US 3250715 A


**Non-patent literature cited in the description**


- Friedel-Crafts and Related Reactions. Interscience Publishers, 1963
- *Friedel-Crafts and Related Reactions,* vol. 2
- **KLAMANN.** Lubricants and Related Products. Verlag Chemie
- **M. W. RANNEY.** Lubricant Additives. Noyes Data Corporation of Parkridge, 1973
- **W. W. YAU ; J. J. KIRKLAND ; D. D. BLY.** Modern Size Exclusion Liquid Chromatography. John Wiley and Sons, 1979